# FASCICULE DE BREVET EUROPEEN

(11) **EP 0 787 209 B1**
(45) Date de publication et mention de la délivrance du brevet: **20.03.2002**
(21) Numéro de dépôt: 96926456.3
(22) Date de dépôt: 24.07.1996
(51) Int. Cl.: C12Q 1/68, C12P 19/34

(54) **PROCEDE D'AMPLIFICATION DE SEQUENCES D'ACIDE NUCLEIQUE PAR DEPLACEMENT, A L'AIDE D'AMORCES CHIMERES**
VERFAHREN ZUR AMPLIFIZIERUNG VON NUKLEINSÄUSE SEQUENZEN DURCH VERDRÄNGUNG MIT HILFE VON CHIMÄREN PRIMER
METHOD FOR AMPLIFYING NUCLEIC ACID SEQUENCES BY DISPLACEMENT USING CHIMERIC PRIMERS

(30) Priorité: 24.07.1995 FR 9508945
(43) Date de publication de la demande: 06.08.1997
(73) Titulaire: BIO MERIEUX, 69280 Marcy l'Etoile (FR)
(72) Inventeur: CLEUZIAT, Philippe, F-69003 Lyon (FR); MANDRAND, Bernard, F-69100 Villeurbanne (FR)
(74) Mandataire: Tonnellier, Jean-Claude
(86) Numéro de dépôt international: FR9601166
(87) Numéro de publication internationale: WO9704126

(56) Documents cités:
- EP-A- 0 500 224
- EP-A- 0 667 393
- WO-A-92/00384
- WO-A-93/09250
- WO-A-95/03426
- DATABASE WPI Section Ch, Week 9507 Derwent Publications Ltd., London, GB; Class B04, AN 95-047919 XP002002117 & JP,A,06 327 500 (TOYOBO KK) , 29 Novembre 1994
- PROCEEDINGS OF THE NATIONAL ACADEMY OF SCIENCES OF USA, vol. 89, no. 1, 1 Janvier 1992, pages 392-396, XP000368694 TERRANCE WALKER G: "ISOTHERMAL IN VITRO AMPLIFICATION OF DNA BY A RESTRICTION ENZYME/ DNA POLYMERASE SYSTEM"
- GENOME RES. (1995), 5(4), 400-3 CODEN: GEREFS, Novembre 1995, XP000545923 SHIBATA, HIROKI ET AL: "RNA-primed PCR"

### LISTE DE SEQUENCES

(1) INFORMATIONS GENERALES:
   (i) DEPOSANT:
      (A) NOM: BIO MERIEUX
      (B) RUE: Chemin de l'Orme
      (C) VILLE: MARCY L'ETOILE
      (E) PAYS: FRANCE
      (F) CODE POSTAL: 69280
      (G) TELEPHONE: (33) 78 87 20 00
      (H) TELECOPIE: (33) 78 87 20 90
   (ii) TITRE DE L'INVENTION: Procédé d'amplification de séquences d'acide nucléique par déplacement, à l'aide d'amorces chimères.
   (iii) NOMBRE DE SEQUENCES: 9
   (iv) FORME DECHIFFRABLE PAR ORDINATEUR:
      (A) TYPE DE SUPPORT: Disquette
      (B) ORDINATEUR: PC compatible
      (C) SYSTEME D'EXPLOITATION: MS-DOS #6.21
      (D) LOGICIEL: Word #6.0 pour Windows #3.1
   (vi) DONNEES DE LA DEMANDE ANTERIEURE:
      (A) NUMERO DE LA DEMANDE: FR 95 08945
      (B) DATE DE DEPOT: 24-JUL-1995
(2) INFORMATIONS POUR LA SEQ ID NO: 1:
   (i) CARACTERISTIQUES DE LA SEQUENCE:
      (A) LONGUEUR: 40 paires de bases
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (iv) ANTI-SENS: NON
   (vi) ORIGINE: synthèse chimique
   (xi) DESCRIPTION DE LA SEQUENCE: SEQ ID NO: 1:
   CTCTTACTGT CATGCCATCC GTCTCGTCTC GTCTCGTCTC 40
(2) INFORMATIONS POUR LA SEQ ID NO: 2:
   (i) CARACTERISTIQUES DE LA SEQUENCE:
      (A) LONGUEUR: 40 paires de bases
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (iv) ANTI-SENS: NON
   (vi) ORIGINE: synthèse chimique
   (xi) DESCRIPTION DE LA SEQUENCE: SEQ ID NO: 2:
   ACACTGCGGC CAACTTACTT GTCTCGTCTC GTCTCGTCTC 40
(2) INFORMATIONS POUR LA SEQ ID NO: 3:
   (i) CARACTERISTIQUES DE LA SEQUENCE:
      (A) LONGUEUR: 40 paires de bases
      (B) TYPE: nucléotide
      (C) NOMBRE DE BRINS: simple
      (D) CONFIGURATION: linéaire
   (ii) TYPE DE MOLECULE: autre acide nucléique : chimère ARN-ADN
   (iii) HYPOTHETIQUE: NON
   (iv) ANTI-SENS: NON
   (vi) ORIGINE: synthèse chimique
   (ix) CARACTERISTIQUE:
      (A) NOM/CLE: caractéristique particulière
      (B) EMPLACEMENT:1..20
      (D) AUTRES INFORMATIONS:
         - les nucléotides 1 à 20 sont des ribonucléotides
         - les nucléotides 21 à 40 sont des désoxyribonucléotides
   (xi) DESCRIPTION DE LA SEQUENCE: SEQ ID NO: 3:
   GAGACGAGAC GAGACGAGAC GGATGGCATG ACAGTAAGAG 40
(2) INFORMATIONS POUR LA SEQ ID NO: 4:
   (i) CARACTERISTIQUES DE LA SEQUENCE:
      (A) LONGUEUR: 40 paires de bases
      (B) TYPE: nucléotide
      (C) NOMBRE DE BRINS: simple
      (D) CONFIGURATION: linéaire
   (ii) TYPE DE MOLECULE: autre acide nucléique : chimère ARN-ADN
   (iii) HYPOTHETIQUE: NON
   (iv) ANTI-SENS: NON
   (vi) ORIGINE: synthèse chimique
   (ix) CARACTERISTIQUE:
      (A) NOM/CLE: caractéristique particulière
      (B) EMPLACEMENT:1..20
      (D) AUTRES INFORMATIONS:
         - les nucléotides 1 à 20 sont des ribonucléotides
         - les nucléotides 21 à 40 sont des désoxyribonucléotides
   (xi) DESCRIPTION DE LA SEQUENCE: SEQ ID NO: 4:
   GAGACGAGAC GAGACGAGAC AAGTAAGTTG GCCGCAGTGT 40
(2) INFORMATIONS POUR LA SEQ ID NO: 5:
   (i) CARACTERISTIQUES DE LA SEQUENCE:
      (A) LONGUEUR: 40 paires de bases
      (B) TYPE: nucléotide
      (C) NOMBRE DE BRINS: simple
      (D) CONFIGURATION: linéaire
   (ii) TYPE DE MOLECULE: autre acide nucléique : chimère ADN-ARN-ADN
   (iii) HYPOTHETIQUE: NON
   (iv) ANTI-SENS: NON
   (vi) ORIGINE: synthèse chimique
   (ix) CARACTERISTIQUE:
      (A) NOM/CLE: caractéristique particulière
      (B) EMPLACEMENT: 16..20
      (D) AUTRES INFORMATIONS:
         - les nucléotides 16 à 20 sont des ribonucléotides
         - les autres nucléotides sont des désoxyribonucléotides
   (xi) DESCRIPTION DE LA SEQUENCE: SEQ ID NO: 5:
   GAGACGAGAC GAGACGAGAC GGATGGCATG ACAGTAAGAG 40
(2) INFORMATIONS POUR LA SEQ ID NO: 6:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (iv) ANTI-SENS: NON
   (vi) ORIGINE: synthèse chimique
   (xi) DESCRIPTION DE LA SEQUENCE: SEQ ID NO: 6:
   GAGACGAGAC GAGACGAG 18
(2) INFORMATIONS POUR LA SEQ ID NO: 7:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: ARN
   (iii) HYPOTHETIQUE: NON
   (iv) ANTI-SENS: NON
   (vi) ORIGINE: synthèse chimique
   (xi) DESCRIPTION DE LA SEQUENCE: SEQ ID NO: 7:
   GAGACGAGAC GAGACGAG 18
(2) INFORMATIONS POUR LA SEQ ID NO: 8:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: ARN
   (iii) HYPOTHETIQUE: NON
   (iv) ANTI-SENS: NON
   (vi) ORIGINE: synthèse chimique
   (xi) DESCRIPTION DE LA SEQUENCE: SEQ ID NO: 8:
   GAGACGAGAC GAGACGAGAC 20
(2) INFORMATIONS POUR LA SEQ ID NO: 9:
   (i) CARACTERISTIQUES DE LA SEQUENCE:
      (A) LONGUEUR: 17 paires de bases
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (iv) ANTI-SENS: NON
   (vi) ORIGINE: synthèse chimique
   (xi) DESCRIPTION DE LA SEQUENCE: SEQ ID NO: 9:
   ATAACACTGC GGCCAAC 17

## Description

La présente invention concerne l'amplification de séquences d'acide nucléique. En particulier, la présente invention a pour objet un procédé pour l'amplification d'acides nucléiques, ainsi que les réactifs destinés à la mise en oeuvre de ce procédé.

Il est souvent nécessaire, dans les technologies relatives aux acides nucléiques et au matériel génétique, de déterminer si un gène, une partie de gène ou une séquence nucléotidique est présent chez un organisme vivant, un extrait cellulaire de cet organisme ou un échantillon biologique. Tout gène ou partie de gène étant caractérisé par une séquence spécifique de bases nucléotidiques, il suffit donc de rechercher directement la présence de tout ou partie de ladite séquence spécifique au sein d'un échantillon contenant un mélange de polynucléotides.

L'intérêt de cette recherche de séquences nucléotidiques spécifiques est immense, notamment pour la détection d'organismes pathogènes, la détermination de la présence d'allèles, la détection de la présence de lésions dans un génome hôte ou la détection de la présence d'un ARN particulier ou de la modification d'un hôte cellulaire. Les maladies génétiques telles que la maladie de Huntington, la myopathie de Duchenne, la phénylcétonurie et la β-thalassémie peuvent ainsi être diagnostiquées par le biais de l'analyse des acides nucléiques des individus. De même, il est possible d'effectuer le diagnostic ou l'identification des virus, viroïdes, bactéries, champignons, protozoaires, ou toute autre forme de vie végétale ou animale, par des tests mettant en oeuvre des sondes nucléiques.

Dans les quelques exemples cités précédemment, après avoir identifié une séquence spécifique d'un organisme ou d'une maladie, il convient d'extraire les acides nucléiques d'un échantillon, et de déterminer si cette séquence est présente.

Différents types de méthodes de détection des acides nucléiques sont décrits dans la littérature. Ces méthodes reposent sur les propriétés d'appariement purinepyrimidine des brins complémentaires d'acides nucléiques dans les duplex ADN-ADN, ADN-ARN et ARN-ARN. Ce processus d'appariement s'effectue par l'établissement de liaisons hydrogène entre les bases adénine-thymine (A-T) et guanine-cytosine (G-C) de l'ADN double brin ; des paires de bases adénine-uracile (A-U) peuvent également se former par liaison hydrogène dans les duplex ADN-ARN ou ARN-ARN. L'appariement de brins d'acide nucléique pour la détermination de la présence ou de l'absence d'une molécule d'acide nucléique donnée est communément appelée "hybridation d'acides nucléiques" ou simplement "hybridation".

La méthode la plus directe pour détecter la présence d'une séquence cible dans un échantillon d'acide nucléique est d'obtenir une "sonde" dont la séquence est suffisamment complémentaire d'une partie de l'acide nucléique cible pour s'hybrider à celui-ci. La sonde ainsi synthétisée peut être appliquée dans un échantillon contenant des acides nucléiques. Si la séquence cible est présente, la sonde formera avec la cible un produit d'hybridation. En l'absence de séquence cible, aucun produit d'hybridation ne se formera. Si la sonde synthétisée est couplée à un marqueur détectable, le produit d'hybridation peut être détecté en mesurant la quantité de marqueur présent. Le transfert de type Southern (SOUTHERN E.M., *J. Mol. Biol.*, 98, 503 (1975)) ou Northern ou la technique du Dot blot ou l'hybridation sandwich (DUNN A.R. et HASSEL J. A., *Cell,* 12, 23, (1977)) constituent des exemples où ces méthodes sont utilisées.

La principale difficulté de cette approche est, cependant, qu'elle n'est pas directement applicable aux cas où le nombre de copies de la séquence cible présente dans un échantillon est faible, inférieur à 10⁷ environ. Dans ces conditions, il est difficile de distinguer un signal significatif, supérieur au bruit de fond de la réaction (c'est à dire de distinguer la fixation spécifique d'une sonde sur sa séquence cible de la fixation non spécifique entre la sonde et une séquence différente de la séquence cible). Une des solutions à ce problème consiste à augmenter le signal de détection par une technique préliminaire visant à augmenter considérablement, de manière spécifique, le nombre de copies d'un fragment d'acide nucléique cible, s'il est présent dans l'échantillon. Une telle technique est couramment appelée technique d'amplification.

Les articles de Lewis (1992. *Genetic Engineering News* 12 : 1-9) d'une part, et d'Abramson et Myers (1993. *Curr. Opin. Biotechnol.* 4 : 41-47) d'autre part, constituent de bonnes revues générales des techniques d'amplification. Ces techniques reposent principalement : a) soit sur la répétition de cycles de synthèse d'ADN *in vitro* par élongation d'amorces nucléotidiques hybridées sur la séquence cible à amplifier par une ADN polymérase comme dans la méthode dite PCR ("Polymerase Chain Reaction", brevets des États Unis d'Amérique n°4 683 195, 4 683 202 et 4 800 159 ; brevet Européen n° 0 201 184 ; ou la technique dite RCR ("Repair Chain Reaction"), demande de brevet n° WO 90/01069 ; la méthode d'amplification avec déplacement de brin ("Strand Displacement Amplification", dite SDA), brevet Européen n° 0 497 272 ; ou la méthode d'amplification avec déplacement de brin au moyen d'une exonucléase, "exonuclease-mediated strand displacement amplification" brevet Européen n° 0 500 224) ; b) soit sur la répétition de cycles de synthèse d'ARN *in vitro*, par réaction de transcription, par une ARN polymérase ADN ou ARN dépendante dont l'activité est obligatoirement associée à une région spécifique, dite région promotrice, renfermant une séquence ou une structure jouant le rôle de promoteur (technique dite TAS, demande de brevet n° WO 88/10315; technique dite 3SR ("Self-Sustained Sequence Replication") décrite dans la demande de brevet WO 90/06995 et le brevet Européen n° 0 373 960 ; la technique dite NASBA ("Nucleic Acid Sequence-Based Amplification") décrite dans la demande de brevet WO 91/02818 et le brevet Européen n° 0 329 822 ; la technique dite SPSR ("Single Primer Sequence Replication") décrite dans le brevet des États Unis d'Amérique n° 5 194 370); la méthode de transcription activée par ligation ("Ligation Activated Transcription") dite LAT, brevet des États Unis d'Amérique n° 5 194 370 et demande de brevet européen n°0 369 775).

Néanmoins, toutes les techniques d'amplification précédemment citées possèdent au moins une limitation importante. Elles ne permettent, par exemple, d'obtenir un produit d'amplification qu'à partir d'un seul type d'acide nucléique cible : ARN ou ADN. Un autre inconvénient de certaines techniques d'amplification est la limitation de la taille du produit de réaction d'amplification. Les techniques telles que la RCR ou la LCR ne permettent d'amplifier que la séquence de la cible correspondant aux amorces et aux sondes nucléotidiques utilisées dans le processus d'amplification. Le bruit de fond non spécifique (c'est à dire en absence de la cible) est également un sérieux inconvénient de certaines techniques, ainsi dans le cas de la LCR, une ligation des extrémités des oligonucléotides libres en excès s'effectue en absence de la cible.

Toutefois, le point critique de toutes ces techniques d'amplification réside dans l'étape de dissociation brin matrice / brin amplifié néo-synthétisé. Dans les techniques évoquées précédemment, la première solution proposée consiste, comme dans la PCR ou la RCR, à réaliser de nombreux cycles de température afin de dissocier les produits de réaction de la cible et permettre à ces produits de servir, à leur tour, de cibles. Cet impératif technique limite par conséquent le choix des enzymes utilisables, dans ces procédés d'amplification, aux enzymes thermostables telles que la *Taq Polymérase* dans la PCR ou une ADN ligase thermostable dans la RCR. Par ailleurs, la réalisation de ces cycles successifs de température constitue un inconvénient pour l'automatisation de ces techniques.

La seconde solution proposée (voir les techniques 3SR, TAS, LAT, NASBA ou celle décrite dans la demande de brevet n° EP 369 775) tire profit des systèmes de transcription via une ARN polymérase ADN ou ARN dépendante dont l'activité est obligatoirement associée à une région promotrice. Ces systèmes présentent l'avantage d'être réalisables dans des conditions isothermes car l'étape de transcription libère des ARN simple brin qui à leur tour peuvent servir de cibles. Toutefois, ces techniques d'amplification présentent elles aussi de nombreuses limites. Ainsi, l'installation aux extrémités des séquences cibles à amplifier d'une région promotrice fonctionnelle est requise pour l'étape de transcription, nécessite souvent plusieurs étapes préalables à la réaction de transcription proprement dite, et fait intervenir une cascade d'activités enzymatiques. Il est par conséquent très difficile de rendre ces techniques performantes du fait de la difficulté de parvenir à des conditions réactionnelles satisfaisant simultanément ces quatre ou cinq activités enzymatiques. Par ailleurs, la création d'un promoteur opérationnel peut nécessiter, à chaque cycle, l'installation de deux oligonucléotides par ligation sur la cible ce qui augmente également le nombre d'étapes (voir par exemple la méthode du promoteur mobile décrite dans la demande de brevet EP 0 369 775).

Enfin, la méthode SDA propose l'utilisation de la propriété de déplacement de brin que possèdent certaines polymérases comme moyen de séparation des brins d'acide nucléique matrice et néo-synthétisé. Cette propriété bien connue a fait l'objet de nombreuses publications scientifiques (voir par exemple, Y. Masamute et C.C. Richardson, 1971, J. Biol. Chem. 246, 2692-2701 ; R.L. Lechner *et al*., 1983, J. Biol. Chem. 258, 11174-11184 ; ou R.C. Lundquist et B.M. Olivera, 1982, Cell 31, 53-60). Cette technique d'amplification permet la multiplication isotherme (37°C) d'une séquence d'ADN cible à l'aide d'une enzyme de restriction approprié et d'une ADN polymérase ADN-dépendante dépourvue d'activité exonucléase (Walker *et al*., 1992. *Proc. Natl. Acad. Sci. USA* 89 : 392-396). Elle est basée sur l'hybridation d'amorces oligonucléotidiques comprenant à leur extrémité 5' une séquence de reconnaissance pour une enzyme de restriction. Après hybridation avec la cible, ces amorces sont élongées par l'ADN polymérase en présence d'au moins un nucléotide modifié (5'[α-thio]dNTP). L'ADN double brin généré est soumis à l'action de l'endonucléase de restriction spécifique. La présence de nucléotides modifiés, sur le brin d'ADN néo-synthétisé renfermant la séquence spécifique de l'endonucléase de restriction, empêche la coupure par l'enzyme de ce brin. Ceci permet d'obtenir une coupure monocaténaire spécifique sur l'amorce, laissant intact le brin complémentaire modifié. L'ADN polymérase peut alors utiliser l'extrémité 3' libérée afin de réaliser une élongation le long de la cible demeurée intacte, et libérer un simple brin d'ADN dans le milieu réactionnel grâce à la propriété de déplacement de brin de la polymérase. Ce brin libéré peut à son tour fixer une amorce contenant une séquence de fixation d'enzyme de restriction, et une réaction cyclique est alors obtenue. De même, il est décrit dans la demande de brevet européen 543 612 une méthode de préparation d'acides nucléiques présentant des extrémités définies et pouvant servir ultérieurement dans une méthode d'amplification, notamment la SDA précitée. Une méthode similaire à la SDA utilisant une activité d'exonucléase au lieu d'une endonucléase et appelée "amplification par déplacement de brin au moyen d'une exonucléase" est décrite dans le brevet européen n° 0 500 224.

Ainsi, la demande de brevet n° WO 93/09250 décrit une méthode de détection d'acide nucléique cible utilisant une méthode SDA comprenant l'utilisation d'une première amorce capable de s'hybrider à un acide nucléique cible et immobilisée sur un support solide, d'une deuxième amorce capable de s'hybrider à l'acide nucléique cible dans la direction opposée, les deux amorces comportant chacune un site de restriction, et d'une enzyme de restriction.

Toutefois, les techniques décrites ci-dessus sont elles aussi limitées. D'une part, la séquence cible à amplifier ne doit pas comporter de site de restriction correspondant à l'endonucléase utilisée dans le procédé. Par conséquent, il est indispensable de connaître, sinon la séquence nucléique totale du fragment à amplifier, au moins la carte de restriction dudit fragment. D'autre part, le choix des nucléases de restriction est restreint à celles présentant la capacité de cliver un site de reconnaissance hémiphosphorothioate (c'est à dire comprenant un brin du duplex avec au moins un nucléotide modifié de type phosphorothioate) et, sur un plan plus général, les sites comportant des nucléotides modifiés. Enfin, outre les contraintes liées au choix du nucléotide modifié et aux impératifs de la synthèse chimique, ces procédés d'amplification sont également limités dans leur rendement, puisqu'il est connu que le Km des polymérases pour les nucléotides modifiés est supérieur à celui pour les nucléotides naturels, d'où une plus faible efficacité d'incorporation enzymatique des nucléotides modifiés dans la cible à amplifier.

Compte tenu de ces nombreux inconvénients, il est donc souhaitable de pouvoir disposer de nouvelles méthodes d'amplification.

Il a également été décrit dans la demande de brevet européen n° 0 667 393 A₂, publiée postérieurement à la date de priorité de la présente invention, une méthode pour produire plus d'une copie d'un acide nucléique cible, les produits obtenus étant sensiblement exempts de séquence codée par l'amorce, cette méthode utilisant une ou plusieurs amorces comprenant au moins un segment d'ARN et éventuellement de l'ADN, chaque amorce étant essentiellement complémentaire d'une séquence distincte de l'acide nucléique cible, un catalyseur produisant de l'acide nucléique, et une ribonucléase H qui retire substantiellement ou la totalité des séquences codées par les amorces, et des produits pour régénérer un site de liaison d'amorce, permettant ainsi un nouvel amorçage et la production de plus d'une copie de l'acide nucléique cible.

Avant d'exposer l'invention, on donne ci-après la définition de certains termes utilisés dans sa description.

Dans la présente demande, l'expression "amont" désigne une région située du coté de l'extrémité 5' de l'acide nucléique ou de la séquence polynucléotidique dont il s'agit, et l'expression "aval" désigne une région située du côté de l'extrémité 3' dudit acide nucléique ou de ladite séquence polynucléotidique.

Par séquence "homologue" d'une autre séquence, on désigne une séquence identique à une autre, ou assez identique pour s'hybrider avec une séquence strictement complémentaire de la séquence avec laquelle elle est homologue.

Le terme "hétéroduplex" désigne un hybride ARN/ADN. Le terme "homoduplex" désigne un hybride ADN/ADN ou ARN/ARN.

Une séquence oligonucléotidique est dite "de type ADN" si elle est constituée d'ADN ou s'il s'agit d'une séquence polynucléotidique modifiée possédant, outre les propriétés d'hybridation de brins des acides nucléiques, au moins une autre propriété en commun avec l'ADN. Cette propriété commune dépendra bien entendu de la fonctionnalité de la séquence modifiée : c'est dans l'exercice de cette fonctionnalité que la séquence en question a une propriété en commun avec l'ADN (c'est-à-dire : se comporte comme un ADN).

Une séquence oligonucléotidique est dite "de type ARN" si elle est constituée d'ARN ou s'il s'agit d'une séquence polynucléotidique modifiée possédant, outre les propriétés d'hybridation de brins des acides nucléiques, au moins une autre propriété en commun avec l'ARN, à savoir d'être sensible à la dégradation par la RNase H dans les mêmes conditions que l'ARN. On sait que la RNase H dégrade sélectivement le brin ARN d'un hybride ARN-ADN.

Dans le procédé de l'invention, le produit de départ comprend un premier segment "correspondant" à la séquence à amplifier, ce qui signifie qu'il s'agit soit de ladite séquence à amplifier, soit du brin complémentaire de ladite séquence à amplifier, étant entendu que le procédé fournit de toute façon une amplification des deux brins complémentaires, même lorsque le produit de départ est monobrin. Elle permet d'obtenir un acide nucléique d'intérêt sous forme de monobrin, ce qui facilite son amplification ultérieure.

La capacité de déplacement de brin, qui est bien connue pour certaines polymérases, concerne, entre autres, la synthèse d'ADN par une ADN polymérase ADN-dépendante ou ARN-dépendante. Bien entendu, cette capacité de déplacement de brins est plus efficace lorsque les polymérases concernées n'ont pas d'activité de 5'-3' exonucléase. Cette capacité de déplacement de brin peut être apportée indépendamment des polymérases, comme cela sera précisé ci-après. Par "activité de déplacement de brin", on désigne le phénomène par lequel un agent biologique, chimique ou physique, par exemple une ADN polymérase, provoque la dissociation du complexe formé par une matrice d'acide nucléique appariée avec son brin complémentaire, cette dissociation progressant dans une direction de 5' vers 3' en conjonction avec l'avancement de la synthèse d'un nouveau brin d'acide nucléique complémentaire de la matrice. Le déplacement de brin commence à l'extrémité 5' d'une séquence d'acide nucléique appariée et se propage vers l'aval au fur et à mesure que progresse la synthèse d'acide nucléique immédiatement en amont du site de déplacement. L'acide nucléique néo-synthétisé et l'acide nucléique déplacé ont généralement la même séquence nucléotidique qui est complémentaire du brin d'acide nucléique matrice. L'activité de déplacement de brin peut être apportée par la même enzyme que celle conférant l'activité de synthèse d'acide nucléique, et particulièrement la synthèse d'ADN, ou elle peut être une activité séparée et indépendante. Des ADN polymérases telles que l'ADN polymérase I d'*E*. *coli*, le fragment de Klenow de l'ADN polymérase I, l'ADN polymérase du bactériophage T7 ou T5, la transcriptase inverse du virus HIV ou la transcriptase inverse du MMLV (Moloney Murine Leukemia Virus) sont des enzymes qui possèdent à la fois une activité de polymérase et une activité de déplacement de brin. Des agents tels que les hélicases peuvent être utilisés en conjonction avec des agents inducteurs qui ne possèdent pas d'activité de déplacement de brin, afin de produire l'effet de déplacement de brin, c'est-à-dire le déplacement d'un acide nucléique simultanément à la synthèse d'un acide nucléique de même séquence. De même, des protéines telles que Rec A ou la Single Strand Binding Protein d'*E. coli* ou d'un autre organisme peuvent être utilisées pour produire ou favoriser le déplacement de brin, en conjonction avec d'autres agents inducteurs. Pour plus de détails et une discussion du déplacement de brin, on peut consulter KORNBERG, A. et BAKER, T.A., DNA Replication, 2nd Edition, pp 113-225, Freeman, New York (1992).

Il convient de remarquer que les polymérases utilisées pour effectuer le déplacement de brin peuvent avantageusement être également pourvue d'une activité de ribonucléase H.

Les termes "fragment d'acide nucléique", "segment d'acide nucléique" ou "oligonucléotide" tels qu'utilisés dans la présente demande désignent un fragment d'ADN ou d'ARN naturel, un polynucléotide naturel ou de synthèse, un fragment d'ADN ou d'ARN de synthèse non modifié ou comprenant au moins une base modifiée telles que l'inosine, la méthyl-5-désoxycytidine, la diméthylamino-5-désoxyuridine, la désoxyuridine, la diamino-2,6-purine, la bromo-5-désoxyuridine, la pseudouridine, la pseudoisocytidine ou toute autre base modifiée permettant l'hybridation. Ce polynucléotide peut aussi être modifié au niveau de la liaison internucléotidique (comme par exemple les liaisons phosphorothioate, H-phosphonate, alkyl phosphonate), au niveau du squelette comme par exemple les alpha-oligonucléotides (brevet français n° 2 607 507) ou les PNA (Egholm *et al*., 1992. *J. Am. Chem. Soc.* 114 : 1895-1897). Chacune des modifications peut être prise en combinaison.

Le terme "support solide" tel qu'utilisé ici inclut tous les matériaux sur lesquels peut être immobilisé un fragment d'acide nucléique pour une utilisation dans des tests diagnostiques, en chromatographie d'affinité et dans des processus de séparation. Des matériaux naturels, de synthèse, poreux ou non, magnétiques ou non, modifiés ou non chimiquement peuvent être utilisés comme support solide, notamment les polysaccharides tels que les matériaux de cellulose, par exemple du papier, des dérivés de cellulose tels que l'acétate de cellulose et la nitrocellulose ; des polymères tels que le chlorure de vinyle, polyéthylène, polystyrène, polyacrylate ou copolymères tels que polymère de chlorure de vinyle et de propylène, polymère de chlorure de vinyle et acétate de vinyle ; copolymères à base de styrènes ; des fibres naturelles telles que le coton et des fibres synthétiques telles que le nylon ; des céramiques; de la silice. Les supports utilisés dans la présente invention sont un polymère de polystyrène, un copolymère butadiène-styrène ou un copolymère butadiène-styrène en mélange avec un ou plusieurs polymères ou copolymères choisis parmi le polystyrène, les copolymères styrène-acrylonitrile ou styrène-méthylmétacrylate de méthyle, les polypropylènes, les polycarbonates ou analogues. Avantageusement, le support de la présente invention est un polystyrène ou un copolymère à base de styrène comprenant entre 10 et 90 % en poids de motifs de styrène ou de la silice. Les supports solides selon l'invention peuvent être, sans limitation, sous la forme d'une plaque de microtitration, d'une feuille, d'un cône, d'un tube, d'un puits, de billes ou analogue.

Le terme "amorce" désigne une structure oligonucléotidique simple brin. Ces nucléotides peuvent être des désoxyribonucléotides et/ou des ribonucléotides. Ces nucléotides peuvent être modifiés comme décrit précédemment dans le paragraphe relatif à la définition du terme "fragment d'acide nucléique". Les amorces oligonucléotidiques, une fois hybridées sur une séquence d'acide nucléique (ADN, ARN, ou molécule chimère ADN-ARN) substantiellement complémentaire sont substrats des polymérases. L'extrémité 3'OH de ces amorces peut être élongée, en présence de nucléotides adéquats et d'une polymérase, conduisant à la synthèse d'un brin complémentaire à la séquence matrice sur laquelle est hybridée ladite amorce. Une amorce peut également être constituée par hybridation de l'extrémité d'une séquence d'acide nucléique simple brin sur elle-même, conduisant notamment à la formation de structures en épingle à cheveux ou tige-boucle. Les amorces oligonucléotidiques hybridées sur une séquence d'acide nucléique ont la propriété de fixer à leur extrémité 3'OH les polymérases.

La présente invention fournit un procédé d'amplification d'une séquence d'acide nucléique cible (ARN et/ou ADN) (ainsi que de sa séquence complémentaire) combinant avantageusement l'utilisation d'amorces chimères et la propriété de déplacement de certaines polymérases. La séquence à amplifier peut être quelconque. Ce procédé présente les avantages de pouvoir être mis en oeuvre de façon isotherme, de permettre l'utilisation d'une seule enzyme combinant les trois activités enzymatiques requises (c'est le cas notamment de la réverse transcriptase) et d'être réalisable indifféremment à partir d'une cible ADN ou ARN, même lorsque les extrémités ne sont pas définies. En outre, il n'implique pas l'incorporation de nucléotides modifiés dans les produits d'amplification par les polymérases utilisées.

L'invention a pour objet un procédé, ainsi qu'un nécessaire, tels que définis dans les revendications annexées, en notant que les expressions "première amorce chimère" et "première amorce", "seconde amorce chimère" et "seconde amorce" "première amorce supplémentaire" et "troisième amorce", seconde amorce supplémentaire" et "quatrième amorce", "première amorce additionnelle" et "cinquième amorce", seconde amorce additionnelle et "sixième amorce" sont équivalentes dans la présente demande.

L'invention a donc pour objet un procédé d'amplification d'une séquence d'un acide nucléique cible, ladite séquence comprenant, à partir de son extrémité 5', une région amont et, à partir de son extrémité 3', une région aval, ledit procédé comprenant les étapes consistant :
- à obtenir un polynucléotide monobrin de type ADN comprenant un premier segment correspondant à la séquence cible à amplifier et comprenant en outre un deuxième segment de séquence arbitraire, situé en aval de l'extrémité 3' dudit premier segment, et
- à mettre en contact ledit polynucléotide monobrin, en présence d'un système enzymatique ayant une activité d'ADN polymérase ADN-dépendante, une activité de déplacement de brin et une activité RNAse H, et en présence d'un excès de désoxyribonucléosides triphosphates,
avec un ensemble d'amorces, présentes en excès, comprenant
a) une première amorce chimère comprenant successivement, dans le sens 5' → 3' :
   - un segment de type ARN, de séquence complémentaire d'au moins une partie de la séquence du second segment dudit polynucléotide monobrin, ladite partie contenant l'extrémité 5' dudit second segment,
   - et un segment de type ADN capable de s'hybrider avec au moins une partie de ladite région aval, ladite partie contenant l'extrémité 3' de ladite région aval, et/ou
b) une seconde amorce chimère comprenant successivement, dans le sens 5' → 3' :
   - un segment de type ARN de séquence arbitraire,
   - et un segment de type ADN, homologue d'au moins une partie de ladite région amont, ladite partie contenant l'extrémité 5' de ladite région amont,
étant entendu que :
soit la première amorce contient, en amont du segment de type ARN, un second segment de type ADN de séquence arbitraire, mais dont au moins une partie contenant son extrémité 3' est capable de s'hybrider avec une partie contenant l'extrémité 3' dudit polynucléotide lorsque ledit segment d'ARN est plus court que ledit second segment dudit polynucléotide simple brin,
soit la première amorce ne contient pas un tel second segment de type ADN et dans ce cas, ledit ensemble d'amorces contient alors une troisième amorce dont au moins une partie, contenant l'extrémité 3' de ladite troisième amorce, est capable de s'hybrider avec au moins une partie du second segment dudit polynucléotide simple brin,
et étant entendu que :
   soit la seconde amorce contient, en amont du segment de type ARN, un second segment de séquence arbitraire de type ADN,
   soit la seconde amorce ne contient pas un tel second segment de type ADN et dans ce cas ledit ensemble d'amorces comprend une quatrième amorce dont au moins une partie, contenant l'extrémité 3' de ladite quatrième amorce, est homologue d'au moins une partie de la séquence du segment de type ARN de la seconde amorce.

Comme on le verra dans la discussion ci-après des dessins annexés, les troisième et quatrième amorces peuvent être de type ADN ou de type ARN.

Les produits de départ utilisés dans le procédé qui vient d'être défini peuvent être préparés soit par synthèse ou hémisynthèse, soit selon les différentes "voies d'entrée" qui seront décrites ci-après.

On décrira ci-après, en faisant référence aux dessins annexés, les schémas de fonctionnement du procédé de l'invention avec certains produits de départ et/ou certaines amorces particulières. Il est aisé de vérifier que l'ensemble des produits de départ (cibles) et/ou amorces tels que définis ci-dessus peuvent donner lieu à une réaction d'amplification selon l'invention.

Les divers segments, présents dans les amorces, qui sont susceptibles de s'apparier avec la cible ou les produits qui en dérivent et d'amorcer alors la synthèse d'un brin nucléotidique dans le procédé de l'invention, ont une longueur suffisante pour permettre l'élongation par une polymérase. Cette longueur suffisante peut être déterminée dans chaque cas par de simples expériences de routine. Elle est d'au moins 2 nucléotides et de préférence d'au moins 5 nucléotides. Ces conditions valent notamment pour les segments de type ADN des première et deuxième amorces ; pour les troisième et quatrième amorces ; et pour les cinquième et sixième amorces qui seront définies ci-après. Les mêmes conditions minimum valent bien entendu pour les segments, régions ou zones du polynucléotide cible (et des divers polynucléotides formés au cours du procédé de l'invention) qui sont susceptibles d'hybridation avec ces amorces ou segments d'amorces.

Quant au segment de type ARN des première et seconde amorces, il contient au moins 1 ribonucléotide, en particulier au moins 2 ribonucléotides et par exemple au moins 4 ribonucléotides.

Dans un mode de réalisation particulier de l'invention, et afin de limiter le nombre d'amorces nucléotidiques utilisées dans la présente invention, les première et deuxième amorces chimères peuvent être choisies identiques, ou partiellement identiques. En particulier, leurs segments respectifs de type ARN peuvent être identiques. De même, leurs seconds segments (facultatifs) de type ADN peuvent être identiques, s'ils sont présents. Par ailleurs, les troisième et quatrième amorces peuvent être identiques, et peuvent en particulier être homologues du segment de type ARN des première et/ou seconde amorces.

Selon un mode de réalisation particulier, l'acide nucléique cible d'origine peut être un ADN ou un ARN isolé d'un échantillon biologique. Dans ce cas, il est possible d'obtenir une réaction d'amplification telle que définie précédemment, au départ de l'acide nucléique cible, en ajoutant tous les réactifs nécessaires (amorces, polymérase, etc.) dès le début de la réaction, éventuellement après dénaturation de la cible. Ce mode de réalisation particulier est caractérisé par le fait que, pour obtenir ledit polynucléotide monobrin de type ADN utilisé comme produit de départ dans le procédé défini précédemment, on opère au départ d'un acide nucléique cible contenant la séquence à amplifier et se prolongeant, au-delà de l'extrémité 3' de ladite séquence à amplifier, par un segment polynucléotidique aval contenant une zone oligonucléotidique dite aval, et se prolongeant éventuellement au-delà de l'extrémité 5' de ladite séquence à amplifier, par un segment polynucléotidique amont,

on met en contact ledit acide nucléique cible en présence d'un excès de désoxyribonucléosides triphosphates et en présence :
- dudit système à activités ADN polymérase, déplacement de brin et RNAse H,
- et d'un ensemble d'amorces contenant des amorces telles que définies précédemment et contenant en outre une cinquième amorce (C1) capable de s'hybrider avec ladite zone aval de la cible. On peut également ajouter une sixième amorce (C2) homologue d'une zone oligonucléotidique, dite zone amont, dudit segment polynucléotidique amont, et donc capable de s'hybrider avec une séquence complémentaire de ladite zone amont. Bien entendu, tous les réactifs peuvent être mélangés dès le départ, la réaction d'amplification cyclique faisant suite à l'obtention du polynucléotide simple brin, défini précédemment comme produit de départ, sans mesure opératoire particulière.

Selon un autre mode de réalisation, le produit de départ est un ARN contenant la séquence à amplifier. Dans ce cas, pour obtenir ledit polynucléotide monobrin de type ADN, il suffit de mettre en contact ledit ARN de départ avec lesdites première et seconde amorces et le cas échéant avec lesdites troisième et quatrième amorces.

L'invention a encore pour objet un nécessaire permettant la mise en oeuvre de la méthode d'amplification décrite précédemment, pour la détection d'un acide nucléique cible susceptible d'être présent dans un échantillon.

L'acide nucléique cible peut être isolé à partir d'un échantillon de toute matière de départ suspectée de le contenir. Pour les animaux, et plus particulièrement les mammifères, l'origine de ces matières peut être le sang, la moelle osseuse, la lymphe, les tissus durs (foie, rate, rein, poumon, ovaires, etc.), des crachats, des frottis, les fèces, l'urine, le sperme, etc. D'autres origines de matières de départ peuvent être des plantes, des prélèvements du sol, des aliments, ainsi que toute autre source suspectée de contenir des organismes biologiques.

L'isolement des acides nucléiques de ces matières de départ peut être réalisé de diverses façons connues, qui ne seront pas rappelées ici.

Dès que les acides nucléiques sont isolés, ils peuvent être soumis à une fragmentation sommaire par des moyens tels que le traitement aux ultrasons, afin d'obtenir des fragments de taille inférieure à 10 kilobases. Cela permet alors de faciliter la dénaturation initiale, en particulier dans le cas d'un acide nucléique double brin.

Les fragments d'acide nucléique cible ainsi obtenus sont dénaturés, le cas échéant, afin de les rendre simple brin et de permettre l'hybridation des amorces A1 et C1, et, si l'acide nucléique initial est double brin, A2 et C2 (ou *vice versa*). Les amorces A1, A2, C1 et C2 sont définies ci-après dans la description des schémas représentés sur les dessins annexés. L'augmentation de la température à environ 95°C est préférable pour la dénaturation, mais la séparation des brins peut également être réalisée par augmentation du pH, puis neutralisation du milieu afin de permettre l'hybridation des amorces sur la cible. Avant ou après que les acides nucléiques cibles soient dénaturés, un mélange réactionnel contenant un excès de désoxyribonucléosides triphosphates, d'amorces appropriées et un mélange d'activités enzymatiques ADN polymérase, déplacement de brin et RNAse H est ajouté. Dans le cas ou l'élévation de température est utilisée pour dénaturer les acides nucléiques cibles, à moins d'utiliser des enzymes thermostables, il est préférable d'ajouter les enzymes après dénaturation. Le mélange réactionnel nécessaire à la réalisation de la réaction d'amplification selon l'invention peut également contenir par exemple des polyols tels que le glycérol, le sorbitol et le polyéthylèneglycol ou des agents dénaturants et/ou des solvants tels que le diméthylformamide le diméthylsulfoxyde (DMSO), des agents stabilisateurs tels que l'albumine, des sucres tels que le tréhalose ou le mannose, etc. Ces réactifs peuvent en effet permettre de réduire les réactions d'hybridation non spécifiques qui pourraient engendrer un éventuel bruit de fond.

Les polymérases utilisées dans le procédé de l'invention sont de préférence pourvues d'une activité de déplacement de brin. Cette activité est une propriété bien connue de certaines ADN polymérases (Sambrook *et al*., 1989. Molecular Cloning : A Laboratory Manual, 2nd Edition, pp. 5.33-5.35, Cold Spring Harbor Laboratory, Cold Spring Harbor). Les propriétés des ADN polymérases, et notamment de l'activité de déplacement de brin de certaines d'entre elles, sont détaillées par Kornberg et Baker, DNA Replication, 2nd Edition, pp. 113-225, Freeman, New York (1992). Le déplacement de brin n'est pas une propriété commune à toutes les ADN polymérases, puisque certaines d'entre elles, comme la T4 ADN polymérases, ne sont pas capables de réaliser, seules, le déplacement de brin. L'activité de déplacement de brin a été mise en évidence initialement pour le fragment de Klenow de l'ADN polymérase I d'*Escherichia* *coli* (Masamune et Richardson, 1971. *J*. *Biol. Chem.* 246 : 2692-2701), ce qui confère à cette enzyme la capacité d'initier la réplication d'un acide nucléique à partir de l'extrémité 3'OH d'une césure sur un ADN double brin. Cette activité de déplacement de brin a également été mise en évidence chez des ADN polymérases thermostables telles que la *Tli* ADN polymérase (Kong *et al*., 1993. *J. Biol. Chem.* 268 : 1965-1975). Dans ce cas, il a également été montré que des formes mutées de cette enzyme ne possédant pas d'activité 5'-3' exonucléase possèdent une plus grande capacité de déplacement de brin. Cette activité de déplacement de brin a également été mise en évidence pour la T7 ADN polymérase (Lechner *et al.*, 1983. *J. Biol. Chem.* 258 : 11174-11184) et pour la transcriptase inverse d'HIV (Huber *et al*., 1989. *J*. *Biol. Chem.* 264 : 4669-4678).

De préférence, une ADN polymérase dépourvue d'activité 5'-3' exonucléase est utilisée pour la réalisation du cycle d'amplification selon l'invention, puisque l'efficacité de l'activité de déplacement de brin est supérieure chez des enzymes dépourvues d'une telle activité d'exonucléase. Le fragment de Klenow de l'ADN polymérase I d'*Escherichia coli* constitue un exemple de polymérase dépourvue d'activité 5'-3' exonucléase, de même que des polymérases telles que la T7 ADN polymérase ou la Sequenase (US Biochemical). La T5 ADN polymérase ou la Phi29 ADN polymérase peuvent également être utilisées. Néanmoins, on peut utiliser une ADN polymérase possédant cette activité 5'-3' exonucléase, lorsque celle-ci n'empêche pas la réalisation de la méthode d'amplification. Dans ce cas, le rendement de la réaction d'amplification peut être amélioré par inhibition spécifique de l'activité 5'-3' exonucléase des ADN polymérases dans les conditions de réaction utilisées.

La présente méthode d'amplification nécessite une étape de transcription inverse lorsque le produit de départ est un ARN. Cette étape de conversion, de l'ARN en ADNc, peut notamment se réaliser par l'utilisation d'une transcriptase inverse de type AMV (Avian Myeloblastosis Virus) ou MMLV (Moloney Murine Leukemia Virus) couramment disponibles commercialement. Toute autre enzyme possédant une activité ADN polymérase ARN- et/ou ADN-dépendante peut être utilisée dans la présente invention, pourvu qu'elle possède une activité de déplacement de brin. Dans le cas contraire, l'activité de déplacement de brin peut être conférée par un agent inducteur, une activité de type hélicase ou Rec A. Les propriétés de Rec A, notamment dans le processus de réassociation d'ADN simple brin, de capture de brin ou d'assimilation de brin sont détaillées par Mc Entee et Weinstock dans The Enzymes, vol. XIV, pp 445-470. L'étape de transcription inverse peut par exemple être réalisée à l'aide de l'ADN polymérase I d'*Escherichia coli*, car il a été démontré que cette enzyme possède également une activité ADN polymérase ARN-dépendante (Ricchetti et Buc, 1993. *EMBO* 12 : 387-396), On peut également utiliser dans ce but des ADN polymérases thermostables ARN- et/ou ADN-dépendantes telles que la Taq polymérase ou la *Tth* polymérase ; pour une revue sur les propriétés des ADN polymérases thermostables, voir Rolf *et al*., PCR ; Clinical Diagnostics and Research, pp. 224-258, Springer-Verlag, Heidelberg (1992).

Du fait de l'utilisation des propriétés de la RNAse H et d'amorces chimères telles que décrites, la présente invention ne nécessite pas d'activité endonucléase ou exonucléase afin d'amorcer le déplacement de brins par l'ADN polymérase.

De même, contrairement à diverses méthodes d'amplification, la présente invention ne nécessite ni des cycles de température, ni une dénaturation chimique afin de dissocier le brin néosynthétisé de sa matrice. Dans cette méthode, une seule température peut être employée, dès que la dénaturation initiale de la cible a été effectuée, le cas échéant. Cette température doit être suffisante pour que les conditions d'hybridation qui autorisent une hybridation spécifique des amorces sur leur cible soient suffisamment discriminantes. Cette température peut être située par exemple entre 37°C et 50°C avec des réactifs enzymatiques conventionnels, mais peut être plus élevée (par exemple 50 à 80°C) si l'on a recours à des enzymes thermostables.

On va maintenant décrire certains modes de réalisation particuliers de l'invention, en faisant le cas échéant référence aux dessins annexés. Dans ces dessins, un segment de ligne droite représente un fragment d'acide nucléique de type ADN, et un segment de ligne ondulée représente un fragment d'acide nucléique de type ARN. Une flèche (→) ou une demi-flèche ( 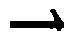 ou 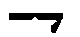 ) symbolise l'extrémité 3'. Le signe // indique l'existence éventuelle d'une partie amont ou aval non représentée. Les couples de brins (ou de brins et segments d'amorces) représentés parallèles sont bien entendu hybridés.

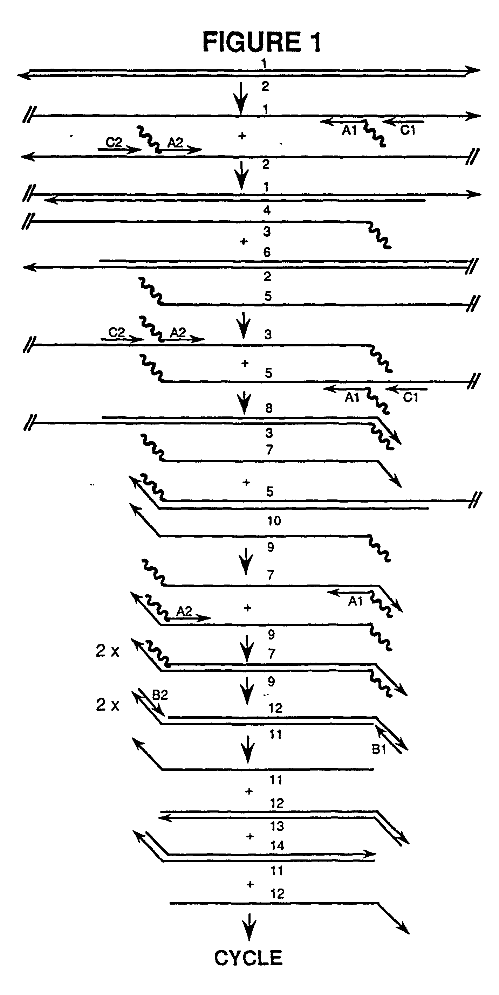

La figure 1 décrit une voie d'entrée pour le procédé d'amplification de l'invention à partir d'ADN, simple ou double brin, utilisant un ensemble d'amorces renfermant deux amorces chimères de type A (A1 et A2) correspondant aux première et seconde amorces, deux amorces de déplacement de type B (B1 et B2) correspondant aux troisième et quatrième amorces et deux autres amorces de déplacement de type C (C1 et C2) correspondant aux cinquième et sixième amorces. Les amorces B et C sont des amorces de séquence de type ADN, tandis que les amorces A sont des amorces chimères telles que définies, et qui dans l'exemple proposé ne contiennent pas de second segment de type ADN.

La figure 2. décrit une variante de la voie d'entrée présentée ci-dessus, utilisant un ensemble d'amorces tel que défini précédemment mais qui contient une seule amorce de déplacement de type C.

La figure 3 décrit une autre variante de la voie d'entrée sur cible ADN, mettant en oeuvre des amorces A renfermant un segment facultatif de type ADN à leurs extrémités 5'.

La figure 4 représente une voie d'entrée de la méthode d'amplification de l'invention, à partir d'une cible d'ARN, utilisant un ensemble d'amorces renfermant seulement deux amorces chimères de type A et deux amorces de déplacement de type B.

La figure 5 représente un schéma réactionnel d'amplification cyclique pouvant être obtenu avec le procédé de l'invention.

La figure 6 représente une variante du schéma réactionnel d'amplification cyclique de la figure 5, dans le cas où les amorces de déplacement B1 et B2 sont constituées d'ARN.

Selon un premier mode de réalisation de l'invention, les amorces A1 et A2 ne renferment pas à leur extrémité 5' la séquence facultative de type ADN. Après dénaturation initiale de l'acide nucléique cible supposé ici sous forme de double brin 1,2, les amorces A1, C1 d'une part et A2, C2 d'autre part s'hybrident sur leur brin d'acide nucléique respectif (figure 1). On notera que la produit de départ de la figure 1 pourrait être un hétéroduplex ADN-ARN. L'élongation simultanée des amorces en présence d'un excès de désoxyribonucléosides triphosphates et d'une ADN polymérase (ARN et/ou ADN dépendante, selon que la cible est de l'ADN ou de l'ARN) conduit au déplacement du brin d'ADN 3 provenant de l'élongation de A1 par élongation du brin 4 issu de C1, et le déplacement du brin 5 provenant de l'élongation de A2 par élongation du brin 6 issu de C2. Sur les ADN simple brin 3 et 5 obtenus par élongation des amorces A1 d'une part et A2 d'autre part, les amorces A2 et C2, et A1 et C1, respectivement peuvent alors hybrider. L'élongation de C1 (produisant le brin 10) provoque le déplacement du brin 9 synthétisé à partir de l'amorce A1, et l'élongation de C2 (produisant le brin 8) provoque le déplacement du brin 7 synthétisé à partir de l'amorce A2. Les deux brins 7 et 9, de longueur définie, ainsi libérés sont parfaitement complémentaires, et peuvent s'hybrider l'un sur l'autre, ou s'hybrider avec les amorces A2 (brin correspondant à l'élongation de A1) ou A1 (brin correspondant à l'élongation de A2), l'hybridation de ces amorces courtes étant cependant favorisée sur le plan thermodynamique.

L'élongation de A1 et A2 sur leurs brins complémentaires respectifs 7 et 9 conduit alors à un polynucléotide chimère double brin (7,9) de longueur définie, chacun des brin consistant en un brin chimère consistant en la séquence cible à amplifier, ou son complémentaire, et comprenant en outre :
- à son extrémité 5', un segment de type ARN de séquence homologue de la séquence du segment de type ARN de l'une ou l'autre des amorces A1 ou A2, selon le brin considéré,
- à son extrémité 3', un segment de type ADN de séquence complémentaire de la séquence du segment de type ARN de l'une ou l'autre des amorces A2 ou A1, selon le brin considéré.

Le polynucléotide chimère double brin obtenu ci-dessus est un substrat de la ribonucléase H (ou RNAse H). La RNAse H permet la dégradation sélective d'un segment de type ARN, dans un hétéroduplex ADN/ARN. Par conséquent, après digestion par la nucléase, on obtient un polynucléotide double brin tel que défini précédemment, dans lequel le segment de type ARN est dégradé sur au moins un des deux brins. Ce polynucléotide double brin 11,12 présente alors à l'extrémité 3' d'au moins l'un de ses brins, une séquence d'ADN, simple brin, qui comprend une séquence complémentaire de l'amorce B1 ou B2, selon le brin considéré. Après hybridation des amorces B1 et/ou B2 sur leur cible respective, ces amorces sont étendues par l'ADN polymerase, provoquant le déplacement du brin d'ADN localisé en amont desdites amorces. On obtient les double brin 12,13 et/ou 11,14, avec libération des produits simple brin 11 et/ou 12 qui renferment la séquence à amplifier (ou son complémentaire) et qui constituent les polynucléotides simple brin pouvant être utilisés comme point de départ du cycle d'amplification selon l'invention.

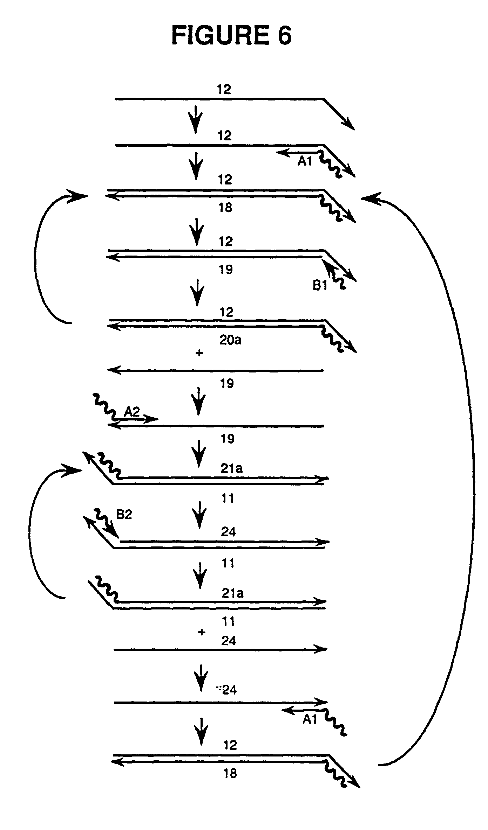

De manière avantageuse, les amorces B1 et/ou B2 sont de type ARN. En effet, dans ce cas, en présence d'un excès d'amorce B1 et/ou B2, comme le montre la figure 6, il est possible d'augmenter le nombre de copies que l'on peut obtenir sur la base d'une seule hybridation de l'amorce A2 et/ou A1. On notera que les brins 7 et 9 peuvent également s'hybrider avec les amorces B1 et B2, respectivement, et on peut aisément vérifier qu'ici encore, on obtiendra la libération des monobrins 11 et 12.

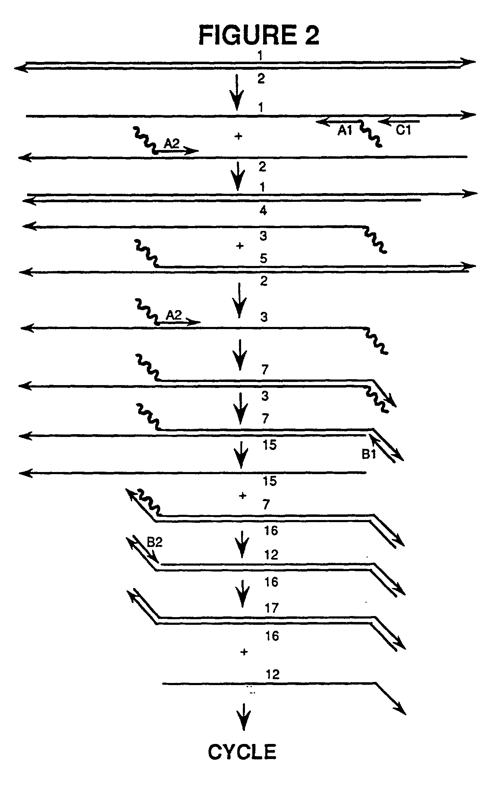

Il est aisé de constater que la voie d'entrée précédemment présentée peut également être réalisée en utilisant seulement l'amorce C1 ou C2, associée aux amorces A1 et A2, et éventuellement B1 et / ou B2. Dans ce cas (Figure 2), après dénaturation initiale de l'acide nucléique cible 1,2, hybridation des amorces A1 et/ou A2 et C1 ou C2, et enfin élongation par l'ADN polymérase, seul le brin 3 provenant de l'élongation de A1 est déplacé par la synthèse du brin 4 issu de C1 (ou bien seul le brin provenant de l'élongation de A2 serait déplacé par élongation du brin issu de C2). Le monobrin 3 peut alors s'hybrider avec l'amorce A2. L'élongation de cette amorce conduit à la formation d'une molécule double brin 3,7, présentant en particulier, à l'une de ses extrémités, un segment hétéroduplex ARN/ADN. La dégradation spécifique de la séquence de type ARN dans cet hétéroduplex par la RNAse H permet d'obtenir à l'extrémité 3' de 7 un segment monobrin de type ADN complémentaire de l'amorce B1, et la transformation du brin 3 en brin 15. L'élongation de cette amorce B1 s'accompagne d'un déplacement du brin 15 et de la production d'une molécule double brin 7,16, présentant elle aussi, à l'une de ses extrémités, un segment hétéroduplex ARN/ADN. Après digestion par la RNAse H, hybridation de l'amorce B2 ou B1, respectivement, et élongation, le polynucléotide simple brin 12, qui renferme la séquence à amplifier, ou son complémentaire, et qui peut être utilisé comme point de départ du cycle d'amplification selon l'invention, est libéré par la formation du duplex 16,17.

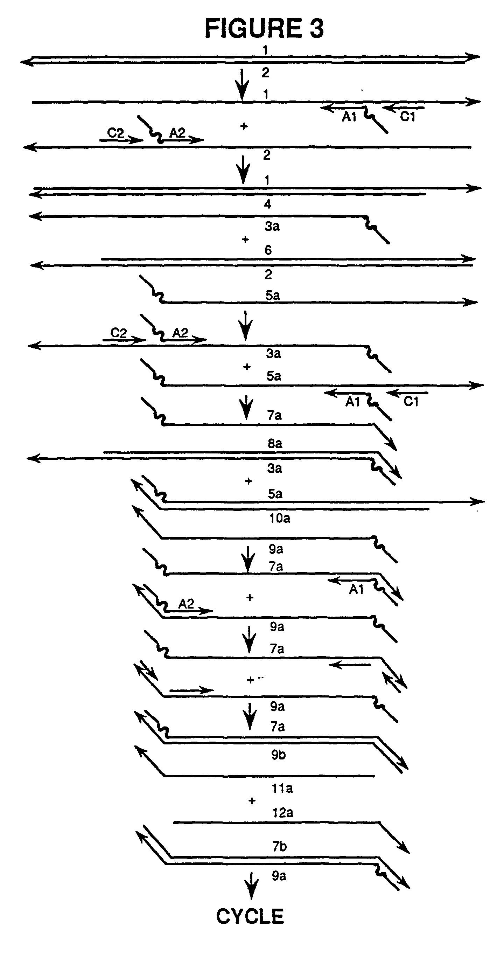

Selon un autre mode de réalisation de l'invention, les amorces A1 et A2 présentent à leur extrémité 5', en amont du segment de type ARN, un segment de type ADN, de séquence définie. Comme le montre la figure 3 cette caractéristique, sans modifier le principe même de l'invention, permet de supprimer l'utilisation des amorces B1 et B2. En effet, lorsque le segment de type ARN de l'amorce A1 ou A2, hybridée à sa cible, est digéré sous l'action de la ribonucléase H, il est aisé de constater que le segment A"1 de type ADN issu de l'amorce A1 (ou A"2 issu de l'amorce A2) peut remplir la fonction de l'amorce de déplacement B1 (ou B2) décrite dans les modes de réalisation précédents.

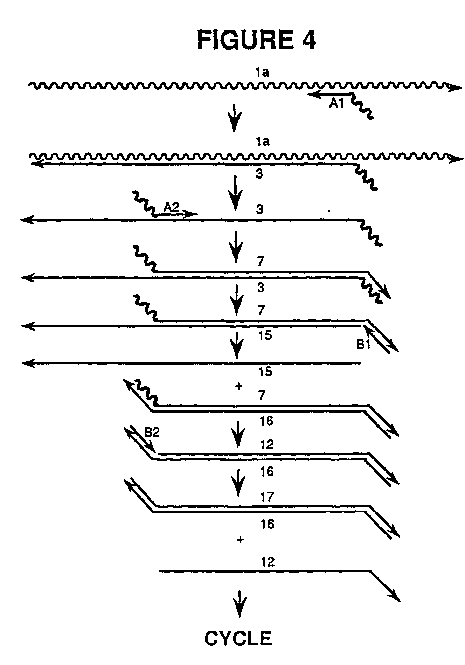

Il est par ailleurs intéressant de noter que lorsque l'acide nucléique de départ est un ARN simple brin, l'utilisation de l'amorce C1 ou C2 n'est plus requise. En effet, comme le montre la figure 4, après hybridation et élongation de l'amorce A1 par exemple le long du brin 1a, la matrice ARN est dégradée par la RNAse H, libérant un monobrin 3 de type ADN qui est capable de s'hybrider avec l'amorce A2. Comme décrit précédemment, l'utilisation alternative des activités de polymérisation associées au déplacement et à la digestion par la RNAse H, en présence des amorces A1 et A2, et éventuellement B1 et/ou B2, permet d'obtenir des polynucléotides simples brins, qui renferment la séquence à amplifier, ou son complémentaire, et qui sont utilisés comme point de départ du cycle d'amplification selon l'invention. On voit sur la figure 4 que le schéma réactionnel devient très rapidement identique à celui de la figure 2. Cette particularité peut être mise en oeuvre lorsque l'on souhaite amplifier de manière spécifique une séquence donnée comprise dans un acide ribonucléique (ARN) et qui est susceptible d'être également comprise, dans le milieu réactionnel d'origine, dans un acide désoxyribonucléique (ADN). Dans ce cas, la seule omission de l'amorce C1 et/ou C2 dans le milieu réactionnel suffit à obtenir une spécificité d'amplification selon la nature, ADN ou ARN, de la cible. Autrement dit, en l'absence de C1 et C2, il sera possible de n'amplifier la séquence cible que si elle est présente sous forme d'ARN dans l'échantillon étudié, et il n'y aura pas d'amplification si ladite séquence cible n'est présente que sous forme d'ADN. On remarquera par ailleurs qu'il est possible d'hybrider sur l'ARN de départ une sonde nucléique de nature ADN afin de définir une extrémité 5' de l'ARN. Grâce à l'hybridation de cette sonde, dite de blocage, et à la digestion par la RNAse H, il est possible de définir précisément jusqu'où l'élongation de l'amorce A1 pourra être réalisée.

Les voies d'entrée évoquées ci-dessus peuvent également être réalisées à partir d'une cible capturée par le biais d'une sonde fixée sur un support solide. Cette sonde peut être immobilisée de manière covalente ou passive, telle que décrite dans le brevet Français n° 91 09057 et la demande de brevet internationale WO 91/19812. Cette immobilisation de sondes peut également être effectuée par le biais de (co)polymères, notamment un copolymère de N-vinylpyrrolidone auxquels sont couplées des sondes par formation d'un conjugué, lequel conjugué est immobilisé sur un support solide par adsorption passive. Plus particulièrement, les amorces A1 et/ou A2, ou C1 et/ou C2 peuvent être fixées sur support solide, à condition que cette fixation ne soit pas réalisée par l'extrémité 3' des amorces, afin que l'extrémité 3' de ces amorces soit capable d'élongation par une ADN polymérase en présence de désoxyribonucléosides triphosphates. En outre, tout ou partie des amorces A1 et C1 d'une part et A2 et C2 d'autre part peuvent être reliées l'une à l'autre par le biais d'un bras de liaison quelconque (de nature hydrocarbonée, nucléotidique ou autre) et ce, à partir de leur extrémité 5', de façon à contrôler et équilibrer les quantités respectives de A1 par rapport à C1, d'une part, et de A2 par rapport à C2, d'autre part, dans la réaction d'amplification décrite.

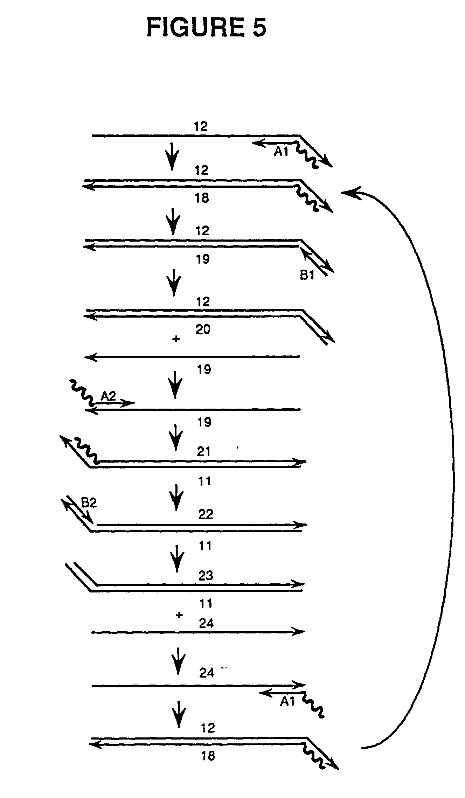

Le polynucléotide simple brin 11 libéré (figure 1) peut s'hybrider avec l'amorce A2. De même, les polynucléotides simple brin tels que 12 ou 12a libérés, comme précédemment, peuvent s'hybrider avec l'amorce A1 (Figure 5). Après élongation de l'amorce par l'ADN polymérase formant le double brin 18,12, et digestion par la ribonucléase H, l'amorce B1 peut à nouveau s'hybrider sur 12 et former le double brin 20,12, en libérant grâce à l'activité de déplacement, la séquence d'ADN simple brin 19 correspondant à la séquence que l'on souhaite amplifier et sur laquelle peut s'hybrider l'amorce A2. Par un schéma analogue utilisant l'amorce B2, on obtient le simple brin 24, et l'élongation de A1 conduit à nouveau au double brin 18,12.

L'ADN issu d'une des deux voies de la méthode d'amplification décrite (figure 5) étant substrat pour la deuxième, et *vice et versa*, il apparaît donc que la méthode selon l'invention est une technique d'amplification cyclique.

On voit sur le schéma de la figure 6 que l'hybridation de A1 sur 12 conduit au double brin 12,19, comme précédemment. L'hybridation de l'amorce B1, qui est cette fois en ARN, conduit au déplacement du brin 19 avec formation du duplex 12,20a qui est semblable ou identique à 12,18. L'hybridation de A2 sur 19 conduit au duplex 11,21a (semblable ou identique à 11,21) et l'action de la RNAse H fournit le duplex 11,24. L'hybridation de la sonde B2 (ARN) libère 24 sous forme de monobrin, avec reformation du duplex 11,21a. L'hybridation de A1 sur 24 conduit au duplex 12,18, et ainsi de suite.

Ainsi, au cours du temps, plusieurs cycles de réaction d'amplification pourront se produire, jusqu'à l'épuisement des réactifs tels que les nucléosides triphosphates et les amorces, conduisant à une amplification dont le rendement correspond à 10⁹ à 10¹² molécules d'ADN produites pour une seule molécule cible initiale. En fonction des concentrations des réactifs utilisés, et notamment des différentes amorces, on peut également privilégier, par la méthode d'amplification, la production de l'un ou l'autre des brins de l'acide nucléique cible de départ.

La mise en oeuvre du procédé de l'invention peut être suivie, si désiré, d'étapes de séparation et/ou de détection des produits de réaction par diverses méthodes connues. Les méthodes de séparation comprennent, entre autres, la séparation magnétique, la capture sur support solide, sur membrane, sur filtre ou sur un polymère. Dans chaque méthode, un résidu de capture peut être fixé à une bille magnétique, à une membrane, à un filtre ou à un polymère. Les billes, la membrane, le support solide, le filtre ou le polymère peuvent être ensuite testés quant à la présence ou l'absence du produit d'amplification. Les résidus de capture peuvent être, par exemple, une séquence d'acide nucléique complémentaire du produit de la réaction d'amplification, des protéines ou des anticorps dirigés contre un ligand ou un haptène incorporé dans une des amorces utilisées, ou dans le produit d'amplification. Le système de séparation peut être couplé ou non au système de détection. Diverses méthodes de détection peut être utilisée. L'une d'entre elles consiste à détecter les produits de réaction ayant une taille définie par séparation électrophorétique. Les méthodes varient selon le procédé de séparation, qui peut impliquer la séparation sur gel, la fixation sur différentes phases solides (billes, plaque de microtitration, latex, particules magnétiques). Une autre méthode utilise le marquage d'une sonde de détection avec un radioisotope tel que le ³² P, par exemple, puis la détection de la radioactivité émise par les produits de réaction en combinaison ou non avec une électrophorèse. Une autre méthode consiste à modifier chimiquement une amorce oligonucléotidique en y ajoutant un ligand (la biotine ou la digoxigénine, par exemple), une enzyme (la phosphatase alcaline, la peroxydase, la β-galactosidase, par exemple), un marqueur fluorescent (la phycobiliprotéine, la fluorescéine ou la rhodamine, par exemple), un marqueur luminescent (un ester d'acridinium, par exemple) ou une combinaison de ces modifications. Une autre méthode consiste à utiliser une amorce nucléotidique de détection qui s'hybridera sur le produit de réaction d'amplification et sera élongée par une polymérase en présence de ribonucléosides triphosphates (cette amorce peut dans ce cas être également modifiée comme décrit précédemment). Les systèmes de détection utiles pour la pratique de l'invention comprennent les systèmes homogènes (c'est à dire qui ne nécessitent pas de système de séparation) et, par opposition, les systèmes hétérogènes. Dans chaque système, un ou plusieurs marqueurs détectables sont utilisés et la réaction ou l'émission du système de détection est mesurée, par exemple par des moyens automatisés. Les exemples de systèmes de détection homogène comprennent le transfert d'énergie de fluorescence, la protection par hybridation (luminescence d'acridinium), la polarisation de fluorescence et la détection immunologique d'un donneur d'enzyme clonée. Les exemples de systèmes hétérogènes comprennent les marqueurs enzymatiques (peroxydase, phosphatase, β-galactosidase), les marqueurs fluorescents (marqueurs enzymatiques, rhodamine, fluorescéine), et les systèmes chimioluminescents et bioluminescents. Dans ces systèmes de détection, les marqueurs détectables peuvent être conjugués à un résidu de capture, ou les produits d'amplification peuvent être générés en présence d'une protéine pouvant être reconnue par un ligand, ou d'un anticorps pouvant être reconnu par un haptène.

Le procédé de l'invention peut également être utilisé comme méthode de détection indirecte d'une molécule d'intérêt dans laquelle un polynucléotide, utilisé comme marqueur couplé à la molécule d'intérêt, est amplifié. Les produits d'amplification du polynucléotide marqueur peuvent eux-mêmes être détectés directement par l'incorporation lors de leur synthèse de nucléotides modifiés tels que marqués au [³² P] ou au [³ H] ou, peuvent être également détectés de façon indirecte selon les méthodes décrites précédemment.

Une autre application du procédé de l'invention est l'obtention d'un produit d'amplification utilisable comme sonde, ou utilisable comme matrice pour la détermination de sa séquence nucléotidique. Les produits amplifiés peuvent être séparés des enzymes utilisées pour l'amplification, afin d'être utilisés dans des procédés ultérieurs impliquant d'autres réactions enzymatiques, d'autres systèmes d'amplification, des méthodes de séquençage ou des méthodes de synthèse d'acides nucléiques, pour ne citer que quelques exemples.

L'invention concerne également un procédé d'amplification d'une séquence d'ADN cible dans lequel on utilise au moins une amorce d'ARN complémentaire d'une zone aval de la séquence cible, en présence de nucléosides triphosphates et d'un système enzymatique ayant une activité d'ADN polymérase avec déplacement et une activité d'RNAse H. L'élongation de l'amorce, suivie de sa digestion par la RNAse, fournit un duplex formé par l'ADN cible et un ADN complémentaire, à extrémité 5' définie. Une nouvelle hybridation de l'amorce sur ce duplex conduit au déplacement dudit brin d'ADN complémentaire à extrémité 5' définie, avec formation du même duplex que précédemment, et ainsi de suite. La figure 6 contient une illustration de ce procédé. Ce procédé peut être utilisé notamment pour obtenir de multiples copies d'un seul brin d'un acide nucléique double brin.

Les exemples suivants illustrent l'invention sans toutefois la limiter. A moins qu'elles ne soient spécifiées, toutes les méthodes relatives à la mise en oeuvre des expériences décrites dans les exemples ci-après ont été réalisées conformément à leur description par Sambrook *et al.* (1989. Molecular Cloning : A Laboratory Manual, 2nd Edition, Cold Spring Harbor Laboratory, Cold Spring Harbor).

### EXEMPLE 1 : Synthèse des oligonucléotides chimères ARN/ADN

Les oligonucléotides chimères RDC-1 (SEQ ID 3 ) et RDC-12 (SEQ ID 4) sont préparés sur synthétiseur Expedite (Millipore). La synthèse se déroule de 3' en 5' en utilisant la chimie des phosphoramidites. La partie ARN est réalisée avec des ribonucléotides phosphoramidite à déprotection rapide fournis par la Société Perkin Elmer. Ils sont protégés en 2' par un groupement ter-butyldiméthylsilyle (t-BDMS), (Admf: réf 401350, Gdmf: réf 401351, Cibu : réf 401352 et U : réf 401353). La partie ADN se situant en 3', la synthèse des chimères démarre sur un support portant un désoxyribonucléotide à déprotection et clivage rapide fournis par la société PerSeptive BiosystemsL Les supports de synthèse sont des colonnes ADN 1µmol DMT-dG(tBPA)-CGP (ref. GEN084184) et thymidine-CPG (ref. GEN061530).

Les deux cycles de synthèse utilisés sont conformes aux instructions du constructeur.

Une solution d'ammoniaque dans l'éthanol est utilisée pour cliver la liaison chimère-support. Cette solution est obtenue en mélangeant 3 volumes d'une solution aqueuse d'ammoniaque (Aldrich, Ref. 338818) à 1 volume d'éthanol pur (Merck, réf. 983).

La déprotection de l'oligonucléotide chimère, à l'exception des groupements hydroxyles en 2' de la partie ARN, est réalisée dans cette même solution ammoniacale pendant 3 heures à 55°C.

Après évaporation de la solution ammoniacale, à l'évaporateur rotatif, 1,5 mL de trifluorhydrate de triéthylamine (TEA 3HF, Aldrich, Réf. 344648) sont ajoutés pour déprotéger les groupements hydroxyles en 2'. Après 24h de contact à température ambiante et sous agitation, avec le TEA 3HF, 300µl d'eau sont ajoutés et les oligonucléotides sont précipités au butanol (Aldrich, Réf. 154679).

Après précipitation, la molécule chimère est séchée sous vide dans un évaporateur rotatif puis reprise dans 1 mL d'eau stérile et purifiée par chromatographie en phase inverse dans les conditions suivantes :
Colonne préparative sur Ultrapore RPMC Beckman dp 5µm, 10*250mm
N° de série n° 238770

- Tampons :: A=Acétate d'ammonium 0,1M pH6.5
B=Acétate d'ammonium 0,05M/Acétonitrile 50%
- Références :: Acétate d'ammonium Merck (ref. 11160500)
- Acétonitrile :: BAKER HPLC gradient grade
- Gradient :: de 0 à 30 % de B en 45 min, avec un débit de 4,7 mL/min.
Les solutions d'oligonucléotides chimères sont chauffées à 90°C pendant 5 minutes puis injectées en HPLC.

### EXEMPLE 2 : Hybridation d'une amorce chimère sur sa cible, digestion par la RNAse H du segment ARN, hybridation d'une amorce de déplacement et élongation de cette amorce

Cet exemple a pour but de montrer d'une part qu'après dégradation par la RNAse H du segment ARN d'une amorce chimère ARN/ADN préalablement hybridée sur une cible ADN, il est possible d'hybrider une amorce (ADN ou ARN) de déplacement en amont du segment ADN, resté hybridé de la chimère, et d'autre part que cette amorce de déplacement peut servir de substrat à une ADN polymérase en vue de son élongation, cette dernière s'accompagnant du déplacement du segment ADN de la chimère. Pour ce faire, plusieurs oligonucléotides ont été utilisés. Dans une première série d'expériences, l'oligonucléotide RDC-6 (SEQ ID No 1) constitue la matrice ADN cible, l'oligonucléotide RDC-1 (SEQ ID No 3) et l'oligonucléotide RDC-4 (SEQ ID No 6) sont les amorces chimère et de déplacement, respectivement. Dans une seconde série, la matrice ADN cible est notée RDC-13 (SEQ ID No 2), l'oligonucléotide chimère RDC-12 (SEQ ID No 4) et l'amorce de déplacement est toujours RDC-4 (SEQ ID No 6). Les séquences des oligonucléotides RDC1 (SEQ ID No 3) et RDC-12 (SEQ ID No 4) sont complémentaires de la séquence des oligonucléotides RDC-6 (SEQ ID No 1) et RDC-13 (SEQ ID No 2), respectivement. La séquence de l'oligonucléotide RDC-4 (SEQ ID No 6) est homologue de la séquence du segment ARN des oligonucléotides RDC1 (SEQ ID No 3) et RDC-12 (SEQ ID No 4). Cet oligonucléotide RDC-4 est radiomarqué au ³² P à son extrémité 5'-OH, par la polynucléotide kinase .

L'oligonucléotide RDC-1 (SEQ ID No 3) et l'oligonucléotide RDC-6 (SEQ ID No 1) ou RDC-12 (SEQ ID No 4) et RDC-13 (SEQ ID No 2), sont mis à incuber pendant 1 minute à 95°C à la concentration de 5.10⁹ copies/ µl chacun dans un volume final de 20 µl de milieu réactionnel 50mM Tris HCl pH 8.3, 75 mM KCl, 3Mm MgCl2, 10 mM DTT et contenant 1mM de chacun des dNTPs. Les tubes sont ensuite placés 10 minutes à 37°C afin de permettre l'hybridation des oligonucléotides avant l'addition éventuelle de 0,8 unités de RNAse H (RNAse thermostable Epicentre Technologies) et/ou de 200 unités de transcriptase inverse (Superscript II, Gibco-BRL). On effectue en parallèle des contrôles réactionnels sans enzyme et/ou sans matrice. Après incubation pendant 15 minutes à 37°C, l'oligonucléotide marqué RDC-4 (SEQ ID No 6) est ajouté au milieu à la concentration de 5.10⁹ copies/ µl. L'incubation à 37°C est poursuivie pendant 30 minutes et la réaction est arrêtée par refroidissement sur de la glace. Une partie de chaque échantillon (10 µl) est mélangée avec 10µl de "bleu formamide" (90% formamide, 0,02% xylène cyanole, 0,02% bleu de bromophénol, 25 mM EDTA) puis analysée par électrophorèse sur gel 15% polyacrylamide - 7M urée en présence d'un marqueur de poids moléculaire formé par un mélange d'oligodésoxyribonucléotides de 70, 60, 40, 33, 25, 20 et 15 nucléotides. Après séchage, le gel est autoradiographié sur film X-Ray.

Quel que soit le jeu d'amorces (RDC-1 et RDC-6 ou RDC-12 et RDC-13), on observe un produit d'élongation dont la taille (40 bases) correspond à l'extension de l'amorce RDC-4 marquée, le long de la cible ADN (RDC-6 ou RDC-13, respectivement) lorsque ladite amorce RDC-4 est hybridée puis élongée. Ce produit n'est observable qu'en présence de la RNAse H et de la réverse transcriptase ; en l'absence de l'une de ces enzymes, aucun produit d'élongation n'est détectable.

Dans une seconde série d'expériences, l'amorce de déplacement de type ADN RDC-4 a été remplacée par l'amorce de déplacement de type ARN RDC-9 (SEQ ID No 7). Les conditions opératoires sont identiques à celles exposées ci-dessus. Les résultats obtenus avec cette amorce RDC-9 de type ARN sont comparables à ceux observés en présence de l'amorce RDC-4 de type ADN.

### EXEMPLE 3 : Utilisation d'une amorce chimère ADN/ARN/ADN

Cet exemple a pour but de montrer que l'utilisation d'une chimère plus complexe présentant de 5' vers 3' un segment ADN, puis un segment ARN et enfin un segment ADN, permet d'éviter l'utilisation d'une amorce de déplacement supplémentaire dans le procédé selon l'invention. Pour ce faire, deux oligonucléotides ont été utilisés. L'oligonucléotide RDC-6 (SEQ ID No 1) constitue la matrice ADN cible, l'oligonucléotide RDC-10 (SEQ ID No 5) est l'amorce chimère. La séquence de l'oligonucléotide RDC10 (SEQ ID No 5) est complémentaire de la séquence de l'oligonucléotide RDC-6 (SEQ ID No 1). L'oligonucléotide RDC-10 est radiomarqué au ³² P à son extrémité 5'-OH, par la polynucléotide kinase .

L'oligonucléotide radiomarqué RDC-10 et l'oligonucléotide RDC-6 sont mis en incubation pendant 1 minute à 95°C à la concentration de 5.10⁹ copies/ µl chacun dans un volume final de 20 µl de milieu réactionnel tel que décrit à l'exemple précédent. Les tubes sont ensuite placés 10 minutes à 37°C afin de permettre l'hybridation des oligonucléotides. Le mélange réactionnel est alors déposé sur gel de polyacrylamide non dénaturant afin de récupérer spécifiquement le duplex issu de l'hybridation des monobrins RDC-10 et RDC-6 et d'éliminer l'oligonucléotide marqué RDC-10 non hybridé. Après extraction du matériel purifié par électrophorèse, puis dialyse et précipitation, les acides nucléiques sont solubilisés dans 20 µl de tampon 50mM Tris HCI pH 8.3, 75 mM KCl, 3mM MgCl2, 10 mM DTT et contenant 1mM de chacun des dNTP. 0,1 unités de RNAse H (RNAse thermostable EPI center technologies) et de 200 unités de transcriptase inverse (Superscript II, Gibco-BRL) sont ensuite ajoutées. On effectue en parallèle des contrôles réactionnels sans enzyme et/ou sans matrice Après incubation pendant 30 minutes à 37°C, la réaction est arrêtée par refroidissement sur de la glace. Une partie de chaque échantillon (10 µl) est mélangée avec 10µl de "bleu formamide" (90% formamide, 0,02% xylène cyanol, 0,02% bleu de bromophénol, 25 mM EDTA) puis analysée par électrophorèse sur gel 15% polyacrylamide - 7M urée en présence d'un marqueur de poids moléculaire formé par un mélange d'oligodésoxyribonucléotides de 70, 60, 40, 33, 25, 20 et 15 nucléotides. Après séchage, le gel est autoradiographié sur film X-Ray.

Les résultats observés permettent de constater que l'oligonucléotide RDC-10 s'hybride effectivement à l'oligonucléotide cible RDC-6 et que ce duplex est un substrat pour la RNAse H (témoin sans transcriptase inverse mais en présence de RNAse H et apparition d'un produit radiomarqué d'environ 15 bases). Par ailleurs, les résultats montrent qu'après dégradation de tout ou partie du segment ARN de la chimère RDC-10, le segment ADN de la chimère demeuré hybridé à l'extrémité 3' de la cible RDC-6 est utilisé comme amorce par la polymérase (on obtient un produit radiomarqué de 40 bases).

### EXEMPLE 4 :

Cet exemple montre dans une première série d'expériences, qu'une amorce ARN hybridée sur une cible ADN peut servir de substrat à une ADN polymérase pour son élongation, puis à la RNAse H pour sa dégradation ; et dans une seconde série d'expériences, que la digestion par le RNAse H du segment ARN compris dans une amorce chimère ADN:ARN préhybridée sur une cible ADN permet l'hybridation sur la cible ADN d'une amorce de déplacement de type ARN dont l'élongation par une ADN polymérase s'accompagne du déplacement du brin d'ADN issu de l'amorce chimère. Ce procédé peut être réitéré un grand nombre de fois par dégradation de la partie ARN du produit d'élongation de l'amorce de déplacement puis hybridation d'une nouvelle amorce, élongation de cette amorce avec déplacement du brin hybridé en aval, et ainsi de suite.

Dans la première série d'expériences, une amorce de déplacement ARN RDC 8 (SEQ ID No.8) hybridée sur la matrice ADN cible RDC 6 (SEQ ID No. 1) sert de substrat au fragment de Klenow de l'ADN polymérase I (USB) dépourvue des activités exonucléases 3'-5' et 5'-3'. 10¹¹ copies de l'oligonucléotide RDC 6 (SEQ ID No.1) sont mises à incuber pendant 5 minutes à 65°C puis 3 minutes à 37°C dans le milieu réactionnel décrit précédemment dans l'exemple 2 en présence de 1 mM dATP, dTTP, dGTP et de 0,1 µM de dCTP contenant 1 µCi de [α-³²P]. Après addition de 2x10¹¹ copies de l'amorce de déplacement ARN RDC 8 (SEQ ID No.8), l'incubation est poursuivie en présence de 10 U de Klenow Exo⁻ (USB) et/ou de 0,2 U de RNAse H thermostable (Epicentre Technologies) pendant 30 minutes à 37°C. La réaction est arrêtée par refroidissement sur de la glace. Une partie de chaque échantillon (10 µl) est mélangée à 10 µl de bleu formamide (90% formamide, 0,02% Xylène Cyanol, 0,02% Bleu de Bromophénol, 25 mM EDTA) puis analysée par électrophorèse sur gel dénaturant 15% polyacrylamide 7 M urée pendant 2 heures sous 350 Volts en présence d'un marqueur de poids moléculaire formé par un mélange d'oligodésoxyribonucléotides de 70, 60, 40, 33, 25, 20 et 15 nucléotides. Le gel est analysé par autoradiographie sur film BioMax (Kodak). On observe en présence de Klenow Exo⁻, un produit d'élongation dont la taille (40 bases) correspond à l'élongation de l'amorce de déplacement RDC 8. L'addition de RNAse H exogène dans le milieu réactionnel permet de visualiser un produit de 20 bases correspondant au produit d'élongation de l'amorce RDC 8 dans lequel le fragment d'ARN a été digéré par la RNAse H.

La deuxième série d'expériences est réalisée sur un hétéroduplex formé par hybridation de 2x10¹¹ copies de l'oligonucléotide chimère RDC 1 (SEQ ID No.3) hybridées sur 10¹¹ copies de matrice ADN cible RDC 6 (SEQ ID No.1), en présence de 2x10¹¹ copies d'amorce de déplacement RDC 8 (SEQ ID No.8), selon des conditions expérimentales identiques à celles décrites précédemment. En présence de la Klenow Exo⁻, aucun produit correspondant à l'élongation de l'amorce de déplacement RDC 8 (SEQ ID No.8) n'est observé car alors le site d'hybridation de l'amorce RDC 8 n'est pas accessible sur la cible ADN RDC 6 (SEQ ID No. 1). Lorsqu'on ajoute au milieu réactionnel 0,2 U de RNAse H, on observe un produit de 20 bases correspondant à la taille du produit d'élongation digéré par le RNAse H. Dans cette expérience, on montre que le segment ARN de l'amorce chimère RDC 1 peut être digéré par la RNAse H, une amorce de déplacement ARN RDC 8 peut venir s'hybrider et être élongée par le Klenow Exo⁻, en déplaçant l'ADN restant de la chimère Cela conduit à la reconstitution du duplex RDC 1:RDC 6. Le phénomène peut être réitéré.

Ainsi, dans le produit d'élongation de la première amorce de déplacement, l'ARN peut à son tour être digéré par la RNAse H, rendant accessible le site d'hybridation pour une autre amorce RDC 8 (en excès). On accumule ainsi des produits de 20 bases radiomarqués, par réitération du processus digestion-hybridation-élongation.

### EXEMPLE 5 :

Dans cet exemple, on étudie la reconstitution d'un hétéroduplex ARN:ADN par élongation d'une amorce ADN sur un oligonucléotide chimère en présence d'une ADN polymérase, la digestion par la RNAse H de la partie ARN du duplex néosynthétisé, puis l'élongation d'une amorce de déplacement ADN s'hybridant sur le site libéré lors de la digestion. Dans cette expérience, l'hétéroduplex ARN:ADN est reconstitué en présence du fragment de Klenow de l'ADN polymérase I d'*E*. *coli*. On mélange 10¹³ copies d'amorce ADN A18 (SEQ ID No.9) à 10¹³ copies de chimère RDC 12 (SEQ ID No. 4) dans 20 µl d'un milieu réactionnel 50 mM Tris-HCI pH 7,5, 10 mM MgCl₂, 1 mM DTT en présence de 1 mM de chaque dNTPs. Les tubes sont placés 3 minutes à 65°C puis 5 minutes à 37°C, avant addition de 5 U de Klenow (Boehringer Mannheim). La réaction s'effectue 30 minutes à 37°C et est arrêtée par refroidissement sur de la glace. Les produits sont purifiés par extraction avec un mélange phénolchloroforme-alcool isoamylique et concentrés par filtration sur une unité Microcon 3 (Amicon). Leur dosage est effectué par absorbance à 260 nm.

On met à incuber 3x10¹² copies des produits obtenus dans le milieu réactionnel 50 mM Tris-HCl (pH 7,5), 10 mM MgCl₂, 1 mM DTT en présence de 1 mM de dARP, dGTP, dTTP et de 0,1 µM de dCTP contenant 0,5 µCi de [α-³²P]dCTP, pendant 3 minutes à 65°C puis 5 minutes à 37°C. L'amorce de déplacement RDC 4 (10¹² copies) est ajoutée à la réaction et l'incubation est poursuivie en présence de 5 U du fragment de Klenow (Boehringer Mannheim) en présence ou en l'absence de 0,2 U de RNAse H thermostable (Epicentre technologies) pendant 30 minutes à 37°C. En parallèle, des témoins d'élongation sont réalisés dans les mêmes conditions, en présence de 10¹² copies d'amorce de déplacement RDC 4 hybridées sur 10¹² copies de cible synthétique RDC 13 (SEQ ID No.2) dont la séquence est complémentaire de la séquence de la chimère RDC 12 (SEQ ID No.4). L'analyse des produits de réaction s'effectue comme décrit dans la première série d'expériences de l'exemple 4. L'analyse autoradiographique des témoins révèle un produit d'élongation dont la taille (40 bases) correspond à l'élongation de l'amorce de déplacement RDC 4 (SEQ ID No.6) sur la cible RDC 13 (SEQ ID No.2). En présence de l'hétéroduplex reconstitué, le même produit d'élongation apparaît de manière intense si la RNAse H et le fragment de Klenow sont présents dans le milieu.

## Revendications

1. Procédé d'amplification d'une séquence d'un acide nucléique cible, ladite séquence comprenant, à partir de son extrémité 5', une région amont et, à partir de son extrémité 3', une région aval,
ledit procédé comprenant les étapes consistant :
- à obtenir un polynucléotide monobrin de type ADN (12) comprenant un premier segment correspondant à la séquence cible à amplifier et comprenant en outre un deuxième segment de séquence arbitraire, situé en aval de l'extrémité 3' dudit premier segment, et
- à mettre en contact ledit polynucléotide monobrin,
en présence d'un système enzymatique ayant une activité d'ADN polymérase ADN-dépendante, une activité de déplacement de brin et une activité RNAse H, et en présence d'un excès de désoxyribonucléosides triphosphates,
avec un ensemble d'amorces, présentes en excès, comprenant :
une première amorce chimère (A₁) comprenant successivement, dans le sens 5' → 3' :
- un segment de type ARN, de séquence complémentaire d'au moins une partie de la séquence du second segment dudit polynucléotide monobrin, ladite partie contenant l'extrémité 5' dudit segment,
- et un segment de type ADN capable de s'hybrider avec au moins une partie de ladite région aval, ladite partie contenant l'extrémité 3' de ladite région aval,
étant entendu que :
soit la première amorce chimère contient, en amont du segment de type ARN, un second segment de type ADN de séquence arbitraire, mais dont au moins une partie contenant son extrémité 3' est capable de s'hybrider avec une partie contenant l'extrémité 3' dudit polynucléotide lorsque ledit segment d'ARN est plus court que ledit second segment dudit polynucléotide simple brin,
soit la première amorce chimère ne contient pas un tel second segment de type ADN et dans ce cas, ledit ensemble d'amorces contient alors une première amorce supplémentaire (B1) dont au moins une partie, contenant l'extrémité 3' de ladite première amorce supplémentaire, est capable de s'hybrider avec au moins une partie du second segment dudit polynucléotide simple brin.

2. Procédé selon la revendication 1, **caractérisé par le fait que** ledit ensemble d'amorces comprend :
une seconde amorce chimère (A₂) comprenant successivement, dans le sens 5' → 3':
- un segment de type ARN de séquence arbitraire,
- et un segment de type ADN, homologue d'au moins une partie de ladite région amont, ladite partie contenant l'extrémité 5' de ladite région amont,
et étant entendu que :
soit la seconde amorce chimère contient, en amont du segment de type ARN, un second segment de séquence arbitraire de type ADN,
soit la seconde amorce chimère ne contient pas un tel second segment de type ADN et dans ce cas ledit ensemble d'amorces comprend une seconde amorce supplémentaire (B₂) dont au moins une partie, contenant l'extrémité 3' de ladite seconde amorce supplémentaire, est homologue d'au moins une partie de la séquence du segment de type ARN de la seconde amorce chimère.

3. Procédé selon l'une quelconque des revendications précédentes, **caractérisé par le fait que** l'une au moins desdites première et seconde amorces chimères ne comporte pas de second segment de type ADN.

4. Procédé selon l'une quelconque des revendications précédentes, **caractérisé par le fait que** ladite première amorce supplémentaire est de type ADN.

5. Procédé selon l'une quelconque des revendications 2 à 4, **caractérisé par le fait que** ladite seconde amorce supplémentaire est de type ADN.

6. Procédé selon l'une quelconque des revendications 1 à 3, **caractérisé par le fait que** ladite première amorce supplémentaire est de type ARN.

7. Procédé selon l'une quelconque des revendications 2 à 3, **caractérisé par le fait que** ladite seconde amorce supplémentaire est de type ARN.

8. Procédé selon l'une quelconque des revendications précédentes, **caractérisé par le fait que** le produit de départ est un acide nucléique contenant la séquence à amplifier et se prolongeant, au-delà de l'extrémité 3' de ladite séquence, par un segment polynucléotidique aval contenant une zone oligonucléotidique dite zone aval, et se prolongeant éventuellement au-delà de l'extrémité 5' de ladite séquence par un segment polynucléotidique amont, **caractérisé par le fait que** pour obtenir ledit polynucléotide monobrin de type ADN, on ajoute une première amorce additionnelle (C1) capable de s'hybrider avec ladite zone aval.

9. Procédé selon la revendication 8, **caractérisé par le fait que** l'on ajoute en outre une deuxième amorce additionnelle (C₂) homologue d'une zone oligonucléotidique dudit segment polynucléotidique amont.

10. Procédé selon l'une quelconque des revendications 2 à 7, **caractérisé par le fait que** le produit de départ est un ARN contenant la séquence à amplifier, et que pour obtenir ledit polynucléotide monobrin de type ADN, on met en contact ledit ARN avec lesdites première et seconde amorces chimères et le cas échéant avec lesdites première et seconde amorces supplémentaires.

11. Nécessaire pour la mise en oeuvre d'un procédé d'amplification d'une séquence d'acide nucléique cible, **caractérisé par le fait qu'**il comprend un système enzymatique ayant une activité de RNAse H et au moins une amorce chimère comprenant successivement, dans le sens 5' → 3' :
- un segment, facultatif, de type ADN,
- un segment de type ARN,
- et un segment de type ADN.

12. Nécessaire selon la revendication 11, **caractérisé par le fait qu'**il contient en outre un système enzymatique ayant une activité d'ADN polymérase ADN-dépendante et une activité de déplacement de brin.

## Claims

1. Method for amplifying a sequence of a target nucleic acid, said sequence comprising, from its 5' end, an upstream region and, from its 3' end, a downstream region,
said method comprising the steps consisting:
- in obtaining a single-stranded polynucleotide of the DNA type (12), comprising a first segment corresponding to the target sequence to be amplified and also comprising a second segment of arbitrary sequence, located downstream of the 3' end of said first segment, and
- in bringing said single-stranded polynucleotide into contact,
in the presence of an enzymatic system having DNA-dependant DNA polymerase activity, strand displacement activity and RNAse H activity, and in the presence of an excess of deoxyribonucleoside triphosphates,
with a set of primers, present in excess, comprising:
a first chimeric primer (A1) comprising successively, in the 5' → 3' direction:
- a segment of the RNA type, of sequence complementary to at least a part of the sequence of the second segment of said single-stranded polynucleotide, said part containing the 5' end of said segment,
- and a segment of the DNA type, capable of hybridizing with at least a part of said downstream region, said part containing the 3' end of said downstream region,
it being understood that:
either the first chimeric primer contains, upstream of the segment of the RNA type, a second segment of the DNA type, of arbitrary sequence, but at least a part of which containing its 3' end is capable of hybridizing with a part containing the 3' end of said polynucleotide when said segment of RNA is shorter than said second segment of said single-stranded polynucleotide,
or the first chimeric primer does not contain such a second segment of the DNA type and, in this case, said set of primers then contains a first supplementary primer (B1), at least a part of which, containing the 3' end of said first supplementary primer, is capable of hybridizing with at least a part of the second segment of said single-stranded polynucleotide.

2. Method according to Claim 1, **characterized in that** said set of primers comprises:
a second chimeric primer (A2) comprising successively, in the 5' → 3' direction:
- a segment of the RNA type, of arbitrary sequence,
- and a segment of the DNA type, homologous to at least a part of said upstream region, said part containing the 5' end of said upstream region,
it being understood that:
either the second chimeric primer contains, upstream of the segment of the RNA type, a second segment of arbitrary sequence of the DNA type,
or the second chimeric primer does not contain such a second segment of the DNA type and, in this case, said set of primers comprises a second supplementary primer (B2), at least a part of which, containing the 3' end of said second supplementary primer, is homologous to at least a part of the sequence of the segment of the RNA type of the second chimeric primer.

3. Method according to any one of the preceding claims, **characterized in that** at least one of said first and second chimeric primers does not comprise a second segment of the DNA type.

4. Method according to any one of the preceding claims, **characterized in that** said first supplementary primer is of the DNA type.

5. Method according to any one of Claims 2 to 4, **characterized in that** said second supplementary primer is of the DNA type.

6. Method according to any one of Claims 1 to 3, **characterized in that** said first supplementary primer is of the RNA type.

7. Method according to either of Claims 2 and 3, **characterized in that** said second supplementary primer is of the RNA type.

8. Method according to any one of the preceding claims, **characterized in that** the starting product is a nucleic acid containing the sequence to be amplified and extending, beyond the 3'end of said sequence, via a downstream polynucleotide sequence containing an oligonucleotide zone termed downstream zone, and optionally extending beyond the 5' end of said sequence via an upstream polynucleotide segment, and **characterized in that**, in order to obtain said single-stranded polynucleotide of the DNA type, a first additional primer (C1), capable of hybridizing with said downstream zone, is added.

9. Method according to Claim 8, **characterized in that** a second additional primer (C2), homologous to an oligonucleotide zone of said upstream polynucleotide segment, is also added.

10. Method according to any one of Claims 2 to 7, **characterized in that** the starting product is an RNA containing the sequence to be amplified, and **in that**, in order to obtain said single-stranded polynucleotide of the DNA type, said RNA is brought into contact with said first and second chimeric primers and, where appropriate, with said first and second supplementary primers.

11. Kit for carrying out a method for amplifying a sequence of a target nucleic acid, **characterized in that** it comprises an enzymatic system having RNAse H activity and at least one chimeric primer comprising successively, in the 5' → 3' direction:
- an optional segment of the DNA type,
- a segment of the RNA type,
- and a segment of the DNA type.

12. Kit according to Claim 11, **characterized in that** it also contains an enzymatic system having DNA-dependant DNA polymerase activity and strand displacement activity.

## Patentansprüche

1. Verfahren zur Amplifikation einer Nukleinsäuresequenz als Ziel, wobei die Sequenz ausgehend von ihrem 5'-Ende einen upstream Bereich und ausgehend von ihrem 3'-Ende einem downstream Bereich enthält, wobei das Verfahren die Schritte umfasst, bestehend aus:
- Erhalten eines einsträngigen DNS-Polynukleotids (12), umfassend ein erstes Segment, entsprechend der zu amplifizierenden Zielsequenz und enthaltend zusätzlich ein zweites Segment willkürlicher Sequenz, das downstream des 3'-Endes des ersten Segments gelegen ist, und
- In-Kontakt-bringen des einsträngigen Polynukleotids in Anwesenheit eines enzymatischen Systems mit einer DNS-abhängigen DNS-Polymeraseaktivität, einer Strangverschiebungsaktivität und einer RNAse H-Aktivität und in Anwesenheit eines Überschusses an Desoxyribonukleosidtriphosphaten, mit einem Satz von Primern, vorhanden im Überschuss, umfassend:
einen ersten chimären Primer (A₁), enthaltend in Reihenfolge in 5'→3'-Richtung:
- ein Segment vom Typ RNS einer Sequenz, die mindestens einem Teil der Sequenz des zweiten Segments des einsträngigen Polynukleotids komplementär ist, wobei dieser Teil das 5'-Ende des Segments enthält,
- und ein DNS-Segment, das mit mindestens einem Teil des downstream Bereiches hybridisieren kann, wobei dieser Teil das 3'-Ende des downstream Bereiches enthält, mit der Maßgabe, dass:
entweder der erste chimäre Primer stromauf vom Segment vom Typ RNS ein zweites DNS-Segment willkürlicher Sequenz enthält, aber wobei mindestens ein Teil, enthaltend sein 3'-Ende, mit einem Teil hybridisieren kann, der das 3'-Ende des Polynukleotids enthält, wenn das RNS-Segment kürzer als das zweite Segment des einsträngigen Polynukleotids ist,
oder der erste chimäre Primer kein solches zweites Segment vom Typ DNS enthält und in diesem Fall der Primersatz dann zusätzlich einen ersten ergänzenden Primer (B1) enthält, wobei mindestens ein Teil, enthaltend das 3'-Ende des ersten ergänzenden Primers, mit mindestens einem Teil des zweiten Segments des einsträngigen Polynukleotids hybridisieren kann.

2. Verfahren gemäß Anspruch 1, **dadurch gekennzeichnet, dass** der Primersatz umfasst:
einen zweiten chimären Primer (A₂), enthaltend in dieser Reihenfolge in 5'→3'-Richtung:
- ein Segment vom Typ RNS mit willkürlicher Sequenz,
- und ein Segment vom Typ DNS, homolog mindestens einem Teil des upstream Bereichs, wobei dieser Teil das 5'-Ende des upstream Bereichs enthält,
und mit der Maßgabe, dass:
entweder der zweite chimäre Primer upstream vom Segment vom Typ RNS ein zweites Segment willkürlicher Sequenz vom Typ DNS enthält,
oder dass der zweite chimäre Primer kein solches zweites Segment vom Typ DNS enthält und dass in diesem Fall der Primersatz einen zweiten ergänzenden Primer (B₂) enthält, von dem mindestens ein Teil, enthaltend das 3'-Ende des zweiten ergänzenden Primers, homolog mindestens einem Teil der Sequenz des Segments vom Typ RNS des zweiten chimären Primers ist.

3. Verfahren gemäß einem der vorstehenden Ansprüche, **dadurch gekennzeichnet, dass** mindestens einer der ersten und zweiten chimären Primer kein zweites Segment vom Typ DNS trägt.

4. Verfahren gemäß einem der vorstehenden Ansprüche, **dadurch gekennzeichnet, dass** der ergänzende erste Primer vom Typ DNS ist.

5. Verfahren gemäß einem der Ansprüche 2 bis 4, **dadurch gekennzeichnet, dass** der zweite ergänzende Primer vom Typ DNS ist.

6. Verfahren gemäß einem der Ansprüche 1 bis 3, **dadurch gekennzeichnet, dass** der erste ergänzende Primer vom Typ RNS ist.

7. Verfahren gemäß einem der Ansprüche 2 bis 3, **dadurch gekennzeichnet, dass** der zweite ergänzende Primer vom Typ RNS ist.

8. Verfahren gemäß einem der vorstehenden Ansprüche, **dadurch gekennzeichnet, dass** das Ausgangsprodukt eine Nukleinsäure ist, enthaltend die zu amplifizierende Sequenz und sich über das 3'-Ende dieser Sequenz hinaus durch eine downstream Polynukleotidsequenz, enthaltend eine oligonukleotidische Zone, bezeichnet als downstream Zone, und gegebenenfalls sich über das 5'-Ende der besagten Sequenz hinaus durch ein polynukleotidisches upstream Segment verlängert, **dadurch gekennzeichnet, dass** man zum Erhalt des einsträngigen Polynukleotids vom Typ DNS einen ersten zusätzlichen Primer (C1) hinzugibt, der mit der downstream Zone hybridisieren kann.

9. Verfahren gemäß Anspruch 8, **dadurch gekennzeichnet, dass** man zusätzlich einen zweiten zusätzlichen Primer (C₂) hinzugibt, homolog einer oligonukleotidischen Zone des polynukleotidischen upstream Segments.

10. Verfahren gemäß einem der Ansprüche 2 bis 7, **dadurch gekennzeichnet, dass** es sich bei dem Ausgangsprodukt um eine RNS handelt, enthaltend die zu amplifizierende Sequenz, und dass man zum Erhalten des einsträngigen Polynukleotids vom Typ DNS die RNS mit den ersten und zweiten chimären Primern und erforderlichenfalls mit den ersten und zweiten ergänzenden Primern in Kontakt bringt.

11. Kit zur Durchführung eines Amplifikationsverfahrens einer Nukleinsäuresequenz als Ziel, **dadurch gekennzeichnet, dass** dieser ein enzymatisches System mit einer RNAse H-Aktivität und mindestens einen chimären Primer umfasst, enthaltend in dieser Reihenfolge in 5'→3'-Richtung:
- ein fakultatives Segment vom Typ DNS,
- ein Segment vom Typ RNS,
- und ein Segment vom Typ DNS.

12. Kit gemäß Anspruch 11, **dadurch gekennzeichnet, dass** er zusätzlich ein enzymatisches System mit einer DNS-abhängigen DNS-Polymerase-Aktivität und einer Strangverschiebungsaktivität enthält.
